# EUROPEAN PATENT SPECIFICATION

(11) **EP 2 938 363 B1**
(45) Date of publication and mention of the grant of the patent: **21.08.2019**
(21) Application number: 13815768.0
(22) Date of filing: 24.12.2013
(51) Int. Cl.: C07K 14/34, C12N 15/73

(54) **METHODS AND COMPOSITIONS RELATING TO CRM197**
VERFAHREN UND ZUSAMMENSETZUNGEN FÜR CRM197
PROCÉDÉS ET COMPOSITIONS SE RAPPORTANT À CRM197

(30) Priority: 27.12.2012 US 201261746366 P
(43) Date of publication of application: 04.11.2015
(73) Proprietor: GlaxoSmithKline Biologicals S.A., 1330 Rixensart (BE); Eidgenössische Materialprüfungs- und Forschungsanstalt, 8600 Dübendorf (CH)
(72) Inventor: IHSSEN, Julian, 9014 St. Gallen (CH); KOWARIK, Michael, 8055 Zürich (CH); THÖNY-MEYER, Linda Christiane, 9053 Teufen (CH)
(74) Representative: Johnston, Caroline Louise
(86) International application number: PCT/EP2013/077968
(87) International publication number: WO 2014/102265

(56) References cited:
- WO-A1-2010/150230
- WO-A2-2011/042516
- ALESSANDRA STEFAN ET AL: "Overexpression and purification of the recombinant diphtheria toxin variant CRM197 in", JOURNAL OF BIOTECHNOLOGY, ELSEVIER SCIENCE PUBLISHERS, AMSTERDAM, NL, vol. 156, no. 4, 15 August 2011 (2011-08-15), pages 245-252, XP028119257, ISSN: 0168-1656, DOI: 10.1016/J.JBIOTEC.2011.08.024 [retrieved on 2011-08-25]

### SEQUENCE LISTING

<110> GlycoVaxyn AG Eidgenossische Materialprufungs-und Forschungsanstalt
<120> METHODS AND COMPOSITIONS RELATING TO CRM197
<130> 103399PC
<140> to be assigned
   <141>
<150> 61/746,366
   <151> 2012-12-27
<160> 6
<170> PatentIn version 3.5
<210> 1
   <211> 7093
   <212> DNA
   <213> Artificial Sequence
<220>
   <223> expression plasmid p932
<400> 1
   gtcgagctag taaaagcatt ttaaataagg aggaataaca catatgaaaa agatttggct      60
   ggcgctggct ggtttagttt tagcgtttag cgccagcgcc gcagatgacg ttgttgacag     120
   cagcaaatcc ttcgttatgg aaaacttctc ctcttatcac ggcaccaaac cgggctatgt     180
   ggacagcatt cagaaaggta tccaaaaacc gaaatctggc acgcagggta actacgatga     240
   cgattggaaa gaattctaca gcaccgacaa caaatatgat gcggccggtt actcagttga     300
   caacgaaaat ccgctgtcgg gcaaagccgg cggtgtggtt aaagtcacgt atccgggcct     360
   gaccaaagtc ctggccctga aagtggataa tgcagaaacc atcaaaaaag aactgggtct     420
   gagcctgacg gaaccgctga tggaacaggt tggcaccgaa gaatttatca aacgcttcgg     480
   cgatggtgcc agtcgtgtcg tgctgtccct gccgttcgca gaaggtagct ctagtgtcga     540
   atatattaac aattgggaac aagcgaaagc cctgtccgtg gaactggaaa tcaactttga     600
   aacccgcggc aaacgtggtc aggatgcgat gtatgaatac atggcacaag cttgcgcggg     660
   taatcgcgtt cgtcgcagcg tcggctcctc actgtcttgt atcaacctgg actgggatgt     720
   tatccgtgat aaaaccaaaa cgaaaatcga aagtctgaaa gaacacggcc cgatcaaaaa     780
   caaaatgagc gaatctccga ataaaacggt gtccgaagaa aaagctaaac agtatctgga     840
   agaattccac caaaccgcac tggaacatcc ggaactgtca gaactgaaaa ccgtcacggg     900
   taccaacccg gtgtttgccg gcgcaaatta cgcagcttgg gctgtgaacg ttgcgcaagt     960
   gattgactcg gaaacggccg ataatctgga aaaaaccacg gcggccctga gtattctgcc    1020
   gggcatcggt tccgtgatgg gtattgccga tggcgcagtt catcacaaca ccgaagaaat    1080
   tgtcgcccag tctatcgcac tgtcgagcct gatggttgct caagcgattc cgctggttgg    1140
   cgaactggtt gatatcggct ttgcagctta caacttcgtg gaaagtatca tcaacctgtt    1200
   tcaggttgtc cataactcat ataatcgccc ggcctactcg ccgggtcaca aaacccaacc 1260
   gttcctgcat gacggctacg cggttagctg gaatacggtc gaagattcta ttatccgtac 1320
   cggctttcag ggtgaatctg gccacgacat taaaatcacg gctgaaaaca ccccgctgcc 1380
   gattgccggt gttctgctgc cgaccatccc gggtaaactg gatgtgaata aatcaaaaac 1440
   ccatatctcg gttaacggtc gcaaaattcg tatgcgctgc cgtgcgatcg acggcgatgt 1500
   gaccttctgt cgtccgaaaa gcccggtcta tgtgggcaac ggtgttcatg ctaatctgca 1560
   cgtcgcgttt catcgctcta gttccgaaaa aatccacagt aacgaaatct catcggactc 1620
   cattggtgtg ctgggctacc agaaaacggt ggatcatacc aaagttaata gcaaactgtc 1680
   actgttcttc gaaatcaaat caggctcgca tcatcatcat caccactaat ctagaggatc 1740
   cccgggtacc gagctcgaat tcttgaagac gaaagggcct cgtgatacgc ctatttttat 1800
   aggttaatgt catgataata atggtttctt agacgtcagg tggcactttt cggggaaatg 1860
   tgcgcggaac ccctatttgt ttatttttct aaatacattc aaatatgtat ccgctcatga 1920
   gacaataacc ctgataaatg cttcaataat attgaaaaag gaagagtatg agtattcaac 1980
   atttccgtgt cgcccttatt cccttttttg cggcattttg ccttcctgtt tttgctcacc 2040
   cagaaacgct ggtgaaagta aaagatgctg aagatcagtt gggtgcacga gtgggttaca 2100
   tcgaactgga tctcaacagc ggtaagatcc ttgagagttt tcgccccgaa gaacgttttc 2160
   caatgatgag cacttttaaa gttctgctat gtggcgcggt attatcccgt gttgacgccg 2220
   ggcaagagca actcggtcgc cgcatacact attctcagaa tgacttggtt gagtactcac 2280
   cagtcacaga aaagcatctt acggatggca tgacagtaag agaattatgc agtgctgcca 2340
   taaccatgag tgataacact gcggccaact tacttctgac aacgatcgga ggaccgaagg 2400
   agctaaccgc ttttttgcac aacatggggg atcatgtaac tcgccttgat cgttgggaac 2460
   cggagctgaa tgaagccata ccaaacgacg agcgtgacac cacgatgcct gcagcaatgg 2520
   caacaacgtt gcgcaaacta ttaactggcg aactacttac tctagcttcc cggcaacaat 2580
   taatagactg gatggaggcg gataaagttg caggaccact tctgcgctcg gcccttccgg 2640
   ctggctggtt tattgctgat aaatctggag ccggtgagcg tgggtctcgc ggtatcattg 2700
   cagcactggg gccagatggt aagccctccc gtatcgtagt tatctacacg acggggagtc 2760
   aggcaactat ggatgaacga aatagacaga tcgctgagat aggtgcctca ctgattaagc 2820
   attggtaact gtcagaccaa gtttactcat atatacttta gattgattta aaacttcatt 2880
   tttaatttaa aaggatctag gtgaagatcc tttttgataa tctcatgacc aaaatccctt 2940
   aacgtgagtt ttcgttccac tgagcgtcag accccgtaga aaagatcaaa ggatcttctt 3000
   gagatccttt ttttctgcgc gtaatctgct gcttgcaaac aaaaaaacca ccgctaccag 3060
   cggtggtttg tttgccggat caagagctac caactctttt tccgaaggta actggcttca 3120
   gcagagcgca gataccaaat actgtccttc tagtgtagcc gtagttaggc caccacttca 3180
   agaactctgt agcaccgcct acatacctcg ctctgctaat cctgttacca gtggctgctg 3240
   ccagtggcga taagtcgtgt cttaccgggt tggactcaag acgatagtta ccggataagg 3300
   cgcagcggtc gggctgaacg gggggttcgt gcacacagcc cagcttggag cgaacgacct 3360
   acaccgaact gagataccta cagcgtgagc tatgagaaag cgccacgctt cccgaaggga 3420
   gaaaggcgga caggtatccg gtaagcggca gggtcggaac aggagagcgc acgagggagc 3480
   ttccaggggg aaacgcctgg tatctttata gtcctgtcgg gtttcgccac ctctgacttg 3540
   agcgtcgatt tttgtgatgc tcgtcagggg ggcggagcct atggaaaaac gccagcaacg 3600
   cggccttttt acggttcctg gccttttgct ggccttttgc tcacatgttc tttcctgcgt 3660
   tatcccctga ttctgtggat aaccgtatta ccgcctttga gtgagctgat accgctcgcc 3720
   gcagccgaac gaccgagcgc agcgagtcag tgagcgagga agcggaagag cgcctgatgc 3780
   ggtattttct ccttacgcat ctgtgcggta tttcacaccg catggtgcac tctcagtaca 3840
   atctgctctg atgccgcata gttaagccag tatacactcc gctatcgcta cgtgactggg 3900
   tcatggctgc gccccgacac ccgccaacac ccgctgacgc gccctgacgg gcttgtctgc 3960
   tcccggcatc cgcttacaga caagctgtga ccgtctccgg gagctgcatg tgtcagaggt 4020
   tttcaccgtc atcaccgaaa cgcgcgaggc agctgcggta aagctcatca gcgtggtcgt 4080
   gaagcgattc acagatgtct gcctgttcat ccgcgtccag ctcgttgagt ttctccagaa 4140
   gcgttaatgt ctggcttctg ataaagcggg ccatgttaag ggcggttttt tcctgtttgg 4200
   tcactgatgc ctccgtgtaa gggggatttc tgttcatggg ggtaatgata ccgatgaaac 4260
   gagagaggat gctcacgata cgggttactg atgatgaaca tgcccggtta ctggaacgtt 4320
   gtgagggtaa acaactggcg gtatggatgc ggcgggacca gagaaaaatc actcagggtc 4380
   aatgccagcg cttcgttaat acagatgtag gtgttccaca gggtagccag cagcatcctg 4440
   cgatgcagat ccggaacata atggtgcagg gcgctgactt ccgcgtttcc agactttacg 4500
   aaacacggaa accgaagacc attcatgttg ttgctcaggt cgcagacgtt ttgcagcagc 4560
   agtcgcttca cgttcgctcg cgtatcggtg attcattctg ctaaccagta aggcaacccc 4620
   gccagcctag ccgggtcctc aacgacagga gcacgatcat gcgcacccgt ggccaggacc 4680
   caacgctgcc cgagcgtcaa cggcgccaga tacagcaaac ggctgcgggg gaaatacgcg 4740
   gttaaacgat cgactgccgc tttgccgctg cgccacagcc gccagcatag ccagcctccg 4800
   acccacagca gcaacgccgt cgccagcagc agccatttga aatctccgct ctgcatatcg 4860
   gaaggaatat cgattgccgc tcccgccaga atgcccggca ggaaataaaa cggcggccac 4920
   agcaaacagc caatcaagtt cggcccaata aatttcgcca cgggaagatc cagcatccct 4980
   gccaccatcg gcaccagcgg cctcgtcgga ccgacaaaac gtccgaccag gatcgtgaac 5040
   atactgtgct gatgcagcgc gtgttcggtt ttatccagca gcgacttgtt ctttttcata 5100
   aaagaccagc ggtgtagcgg ctttttaaag cgccacccca gccagaacga aatccagtcg 5160
   cccatcagac agccgataat acccaccagc caggcatgcc aaaaattgag ctcgccgctg 5220
   ccgataagcg cgcccagccc cgccatcagt accgtgccgg gtaaaatcaa ccccaccagc 5280
   gccagcgatt ccaggaaggc gaccagcaac acggcgatga gcgaatacag agtggattgg 5340
   gtgataaagt gttccagcag tgcttgcata gtgtgtccgt cagcgtgatg aagcagggat 5400
   tctgcttacc ccgtccccct tcgtcaagcc gtcaattatc cgaatagtta cggcttatga 5460
   catctttgtg gacacatcat tcacttttta ttcacatccg gccctgaact cgctaggact 5520
   tgccccggtg cattttttaa atacccgcga aaaatagagc tgatcgtcaa atccaacatt 5580
   gcgcccaacg gtcgctatcg gcattcgcgt agtgctaagc agaagtttcg cctggctgat 5640
   acgctgatct tcgcgccagc tcaatacgct aatgcctaac tgctggcgga acagatgtga 5700
   taaccgggag ggcgacaggc agacatgctg ggcgacgctg gcgatatcaa aatggctgtc 5760
   cgccagatgg tcgctgatat actggcaggc atcgcgcaca cggctatcca tcggcgggtg 5820
   caacgactca ttaattaccg ccatacgtct gagcaacaac tgctccagca gattgatcgc 5880
   cagtagctca gaatagcgac cttccccttg cccggcgctg atgatctgcc cgaacagttc 5940
   gctgaaatgc ggctggcgcg cctcgtccgg gcggaaaaat cctgtctggg caaagattgt 6000
   cggccaggtc agccactcct gccagtaggc gcgaggccgg aaataaaccc actggtgata 6060
   ccactcgctg gcgtccggat gccgtccata gtgatgaatc tcgcccggcg gaaacaataa 6120
   tatatcgcca ggccgacaga caaactgctc gccattatta ttaatgacgc cctctccgcg 6180
   gatggtcagg ttaagaatat atcccttcat gcccaacgga cgatcgataa aaaaatccag 6240
   atatccattc gcttcaattg gcgtcagccc ggcgaccaga tgggcattaa atgaatatcc 6300
   cggcaatagc ggatcatttt gcgtttcagc catgatttct ctaccccccg atgttcagag 6360
   aagaaacaaa ttgtccatat cgaccaggac gacagagctt ccgtctccgc aagactttgc 6420
   gcttgatgaa agcacgtatc aaccccgctt gtgaaaagcg ctttgtaaca aaagcgtaca 6480
   gttcaggcga taaaattaag taacagaagt gtctataact atggctggaa tgtccacatt 6540
   gaatatttgc acagcgtcac actttgcaaa gcattagcat ttttgtccat aagattagcg 6600
   gatcctgcct gacggttttt gccgcgactc tctactgttt ctccatacct gtttttctgg 6660
   atggagtaag acgatggcaa ttgcaattgg cctcgatttt ggcagtgatt cagtgcgcgc 6720
   tctggcagtg gactgcgcca ccggcgacga gatcgccacc agcgtagagt ggtatccgcg 6780
   ctggcaagaa ggccgttatt gcgacggccc gaacaaccag ttccgtcatc atccgcgcga 6840
   ctacatggag tcaatggagg ccgcgctgaa agccgttctg gcacaattaa gcgccgcgca 6900
   acgcgcaaat gtcgttggca ttggcgttga cagcaccggc tctacgccag cgccgattga 6960
   cgccgacggt aacgtcctgg cgctgcgtcc agagttcgcc gagaacccga atgcgatgtt 7020
   tgtgctgtgg aaagatcaca ccgccgtgga agaggccgac gaaatcactc gtctgtgcca 7080
   taagccaggc aag 7093
<210> 2
   <211> 7096
   <212> DNA
   <213> Artificial Sequence
<220>
   <223> expression plasmid p933
<400> 2
   gtcgagctag taaaagcatt ttaaataagg aggaataaca catatgaaaa agatttggct     60
   ggcgctggct ggtttagttt tagcgtttag cgccatggca ggcgcagatg acgttgttga    120
   cagcagcaaa tccttcgtta tggaaaactt ctcctcttat cacggcacca aaccgggcta    180
   tgtggacagc attcagaaag gtatccaaaa accgaaatct ggcacgcagg gtaactacga    240
   tgacgattgg aaagaattct acagcaccga caacaaatat gatgcggccg gttactcagt    300
   tgacaacgaa aatccgctgt cgggcaaagc cggcggtgtg gttaaagtca cgtatccggg    360
   cctgaccaaa gtcctggccc tgaaagtgga taatgcagaa accatcaaaa aagaactggg    420
   tctgagcctg acggaaccgc tgatggaaca ggttggcacc gaagaattta tcaaacgctt    480
   cggcgatggt gccagtcgtg tcgtgctgtc cctgccgttc gcagaaggta gctctagtgt    540
   cgaatatatt aacaattggg aacaagcgaa agccctgtcc gtggaactgg aaatcaactt    600
   tgaaacccgc ggcaaacgtg gtcaggatgc gatgtatgaa tacatggcac aagcttgcgc    660
   gggtaatcgc gttcgtcgca gcgtcggctc ctcactgtct tgtatcaacc tggactggga    720
   tgttatccgt gataaaacca aaacgaaaat cgaaagtctg aaagaacacg gcccgatcaa    780
   aaacaaaatg agcgaatctc cgaataaaac ggtgtccgaa gaaaaagcta aacagtatct    840
   ggaagaattc caccaaaccg cactggaaca tccggaactg tcagaactga aaaccgtcac    900
   gggtaccaac ccggtgtttg ccggcgcaaa ttacgcagct tgggctgtga acgttgcgca    960
   agtgattgac tcggaaacgg ccgataatct ggaaaaaacc acggcggccc tgagtattct   1020
   gccgggcatc ggttccgtga tgggtattgc cgatggcgca gttcatcaca acaccgaaga   1080
   aattgtcgcc cagtctatcg cactgtcgag cctgatggtt gctcaagcga ttccgctggt   1140
   tggcgaactg gttgatatcg gctttgcagc ttacaacttc gtggaaagta tcatcaacct   1200
   gtttcaggtt gtccataact catataatcg cccggcctac tcgccgggtc acaaaaccca   1260
   accgttcctg catgacggct acgcggttag ctggaatacg gtcgaagatt ctattatccg   1320
   taccggcttt cagggtgaat ctggccacga cattaaaatc acggctgaaa acaccccgct   1380
   gccgattgcc ggtgttctgc tgccgaccat cccgggtaaa ctggatgtga ataaatcaaa   1440
   aacccatatc tcggttaacg gtcgcaaaat tcgtatgcgc tgccgtgcga tcgacggcga   1500
   tgtgaccttc tgtcgtccga aaagcccggt ctatgtgggc aacggtgttc atgctaatct   1560
   gcacgtcgcg tttcatcgct ctagttccga aaaaatccac agtaacgaaa tctcatcgga   1620
   ctccattggt gtgctgggct accagaaaac ggtggatcat accaaagtta atagcaaact   1680
   gtcactgttc ttcgaaatca aatcaggctc gcatcatcat catcaccact aatctagagg   1740
   atccccgggt accgagctcg aattcttgaa gacgaaaggg cctcgtgata cgcctatttt   1800
   tataggttaa tgtcatgata ataatggttt cttagacgtc aggtggcact tttcggggaa   1860
   atgtgcgcgg aacccctatt tgtttatttt tctaaataca ttcaaatatg tatccgctca   1920
   tgagacaata accctgataa atgcttcaat aatattgaaa aaggaagagt atgagtattc 1980
   aacatttccg tgtcgccctt attccctttt ttgcggcatt ttgccttcct gtttttgctc 2040
   acccagaaac gctggtgaaa gtaaaagatg ctgaagatca gttgggtgca cgagtgggtt 2100
   acatcgaact ggatctcaac agcggtaaga tccttgagag ttttcgcccc gaagaacgtt 2160
   ttccaatgat gagcactttt aaagttctgc tatgtggcgc ggtattatcc cgtgttgacg 2220
   ccgggcaaga gcaactcggt cgccgcatac actattctca gaatgacttg gttgagtact 2280
   caccagtcac agaaaagcat cttacggatg gcatgacagt aagagaatta tgcagtgctg 2340
   ccataaccat gagtgataac actgcggcca acttacttct gacaacgatc ggaggaccga 2400
   aggagctaac cgcttttttg cacaacatgg gggatcatgt aactcgcctt gatcgttggg 2460
   aaccggagct gaatgaagcc ataccaaacg acgagcgtga caccacgatg cctgcagcaa 2520
   tggcaacaac gttgcgcaaa ctattaactg gcgaactact tactctagct tcccggcaac 2580
   aattaataga ctggatggag gcggataaag ttgcaggacc acttctgcgc tcggcccttc 2640
   cggctggctg gtttattgct gataaatctg gagccggtga gcgtgggtct cgcggtatca 2700
   ttgcagcact ggggccagat ggtaagccct cccgtatcgt agttatctac acgacgggga 2760
   gtcaggcaac tatggatgaa cgaaatagac agatcgctga gataggtgcc tcactgatta 2820
   agcattggta actgtcagac caagtttact catatatact ttagattgat ttaaaacttc 2880
   atttttaatt taaaaggatc taggtgaaga tcctttttga taatctcatg accaaaatcc 2940
   cttaacgtga gttttcgttc cactgagcgt cagaccccgt agaaaagatc aaaggatctt 3000
   cttgagatcc tttttttctg cgcgtaatct gctgcttgca aacaaaaaaa ccaccgctac 3060
   cagcggtggt ttgtttgccg gatcaagagc taccaactct ttttccgaag gtaactggct 3120
   tcagcagagc gcagatacca aatactgtcc ttctagtgta gccgtagtta ggccaccact 3180
   tcaagaactc tgtagcaccg cctacatacc tcgctctgct aatcctgtta ccagtggctg 3240
   ctgccagtgg cgataagtcg tgtcttaccg ggttggactc aagacgatag ttaccggata 3300
   aggcgcagcg gtcgggctga acggggggtt cgtgcacaca gcccagcttg gagcgaacga 3360
   cctacaccga actgagatac ctacagcgtg agctatgaga aagcgccacg cttcccgaag 3420
   ggagaaaggc ggacaggtat ccggtaagcg gcagggtcgg aacaggagag cgcacgaggg 3480
   agcttccagg gggaaacgcc tggtatcttt atagtcctgt cgggtttcgc cacctctgac 3540
   ttgagcgtcg atttttgtga tgctcgtcag gggggcggag cctatggaaa aacgccagca 3600
   acgcggcctt tttacggttc ctggcctttt gctggccttt tgctcacatg ttctttcctg 3660
   cgttatcccc tgattctgtg gataaccgta ttaccgcctt tgagtgagct gataccgctc 3720
   gccgcagccg aacgaccgag cgcagcgagt cagtgagcga ggaagcggaa gagcgcctga 3780
   tgcggtattt tctccttacg catctgtgcg gtatttcaca ccgcatggtg cactctcagt 3840
   acaatctgct ctgatgccgc atagttaagc cagtatacac tccgctatcg ctacgtgact 3900
   gggtcatggc tgcgccccga cacccgccaa cacccgctga cgcgccctga cgggcttgtc 3960
   tgctcccggc atccgcttac agacaagctg tgaccgtctc cgggagctgc atgtgtcaga 4020
   ggttttcacc gtcatcaccg aaacgcgcga ggcagctgcg gtaaagctca tcagcgtggt 4080
   cgtgaagcga ttcacagatg tctgcctgtt catccgcgtc cagctcgttg agtttctcca 4140
   gaagcgttaa tgtctggctt ctgataaagc gggccatgtt aagggcggtt ttttcctgtt 4200
   tggtcactga tgcctccgtg taagggggat ttctgttcat gggggtaatg ataccgatga 4260
   aacgagagag gatgctcacg atacgggtta ctgatgatga acatgcccgg ttactggaac 4320
   gttgtgaggg taaacaactg gcggtatgga tgcggcggga ccagagaaaa atcactcagg 4380
   gtcaatgcca gcgcttcgtt aatacagatg taggtgttcc acagggtagc cagcagcatc 4440
   ctgcgatgca gatccggaac ataatggtgc agggcgctga cttccgcgtt tccagacttt 4500
   acgaaacacg gaaaccgaag accattcatg ttgttgctca ggtcgcagac gttttgcagc 4560
   agcagtcgct tcacgttcgc tcgcgtatcg gtgattcatt ctgctaacca gtaaggcaac 4620
   cccgccagcc tagccgggtc ctcaacgaca ggagcacgat catgcgcacc cgtggccagg 4680
   acccaacgct gcccgagcgt caacggcgcc agatacagca aacggctgcg ggggaaatac 4740
   gcggttaaac gatcgactgc cgctttgccg ctgcgccaca gccgccagca tagccagcct 4800
   ccgacccaca gcagcaacgc cgtcgccagc agcagccatt tgaaatctcc gctctgcata 4860
   tcggaaggaa tatcgattgc cgctcccgcc agaatgcccg gcaggaaata aaacggcggc 4920
   cacagcaaac agccaatcaa gttcggccca ataaatttcg ccacgggaag atccagcatc 4980
   cctgccacca tcggcaccag cggcctcgtc ggaccgacaa aacgtccgac caggatcgtg 5040
   aacatactgt gctgatgcag cgcgtgttcg gttttatcca gcagcgactt gttctttttc 5100
   ataaaagacc agcggtgtag cggcttttta aagcgccacc ccagccagaa cgaaatccag 5160
   tcgcccatca gacagccgat aatacccacc agccaggcat gccaaaaatt gagctcgccg 5220
   ctgccgataa gcgcgcccag ccccgccatc agtaccgtgc cgggtaaaat caaccccacc 5280
   agcgccagcg attccaggaa ggcgaccagc aacacggcga tgagcgaata cagagtggat 5340
   tgggtgataa agtgttccag cagtgcttgc atagtgtgtc cgtcagcgtg atgaagcagg 5400
   gattctgctt accccgtccc ccttcgtcaa gccgtcaatt atccgaatag ttacggctta 5460
   tgacatcttt gtggacacat cattcacttt ttattcacat ccggccctga actcgctagg 5520
   acttgccccg gtgcattttt taaatacccg cgaaaaatag agctgatcgt caaatccaac 5580
   attgcgccca acggtcgcta tcggcattcg cgtagtgcta agcagaagtt tcgcctggct 5640
   gatacgctga tcttcgcgcc agctcaatac gctaatgcct aactgctggc ggaacagatg 5700
   tgataaccgg gagggcgaca ggcagacatg ctgggcgacg ctggcgatat caaaatggct 5760
   gtccgccaga tggtcgctga tatactggca ggcatcgcgc acacggctat ccatcggcgg 5820
   gtgcaacgac tcattaatta ccgccatacg tctgagcaac aactgctcca gcagattgat 5880
   cgccagtagc tcagaatagc gaccttcccc ttgcccggcg ctgatgatct gcccgaacag 5940
   ttcgctgaaa tgcggctggc gcgcctcgtc cgggcggaaa aatcctgtct gggcaaagat 6000
   tgtcggccag gtcagccact cctgccagta ggcgcgaggc cggaaataaa cccactggtg 6060
   ataccactcg ctggcgtccg gatgccgtcc atagtgatga atctcgcccg gcggaaacaa 6120
   taatatatcg ccaggccgac agacaaactg ctcgccatta ttattaatga cgccctctcc 6180
   gcggatggtc aggttaagaa tatatccctt catgcccaac ggacgatcga taaaaaaatc 6240
   cagatatcca ttcgcttcaa ttggcgtcag cccggcgacc agatgggcat taaatgaata 6300
   tcccggcaat agcggatcat tttgcgtttc agccatgatt tctctacccc ccgatgttca 6360
   gagaagaaac aaattgtcca tatcgaccag gacgacagag cttccgtctc cgcaagactt 6420
   tgcgcttgat gaaagcacgt atcaaccccg cttgtgaaaa gcgctttgta acaaaagcgt 6480
   acagttcagg cgataaaatt aagtaacaga agtgtctata actatggctg gaatgtccac 6540
   attgaatatt tgcacagcgt cacactttgc aaagcattag catttttgtc cataagatta 6600
   gcggatcctg cctgacggtt tttgccgcga ctctctactg tttctccata cctgtttttc 6660
   tggatggagt aagacgatgg caattgcaat tggcctcgat tttggcagtg attcagtgcg 6720
   cgctctggca gtggactgcg ccaccggcga cgagatcgcc accagcgtag agtggtatcc 6780
   gcgctggcaa gaaggccgtt attgcgacgg cccgaacaac cagttccgtc atcatccgcg 6840
   cgactacatg gagtcaatgg aggccgcgct gaaagccgtt ctggcacaat taagcgccgc 6900
   gcaacgcgca aatgtcgttg gcattggcgt tgacagcacc ggctctacgc cagcgccgat 6960
   tgacgccgac ggtaacgtcc tggcgctgcg tccagagttc gccgagaacc cgaatgcgat 7020
   gtttgtgctg tggaaagatc acaccgccgt ggaagaggcc gacgaaatca ctcgtctgtg 7080
   ccataagcca ggcaag 7096
<210> 3
   <211> 561
   <212> PRT
   <213> Artificial Sequence
<220>
   <223> Signal peptide containing Crm197 amino acid sequence expressed from p932
<400> 3
   Met Lys Lys Ile Trp Leu Ala Leu Ala Gly Leu Val Leu Ala Phe Ser
   1               5                   10                  15
   Ala Ser Ala Ala Asp Asp Val Val Asp Ser Ser Lys Ser Phe Val Met
               20                  25                  30
   Glu Asn Phe Ser Ser Tyr His Gly Thr Lys Pro Gly Tyr Val Asp Ser
           35                  40                  45
   Ile Gln Lys Gly Ile Gln Lys Pro Lys Ser Gly Thr Gln Gly Asn Tyr
       50                  55                  60
   Asp Asp Asp Trp Lys Glu Phe Tyr Ser Thr Asp Asn Lys Tyr Asp Ala
   65                  70                  75                  80
   Ala Gly Tyr Ser Val Asp Asn Glu Asn Pro Leu Ser Gly Lys Ala Gly
                   85                  90                  95
   Gly Val Val Lys Val Thr Tyr Pro Gly Leu Thr Lys Val Leu Ala Leu
               100                 105                 110
   Lys Val Asp Asn Ala Glu Thr Ile Lys Lys Glu Leu Gly Leu Ser Leu
           115                 120                 125
   Thr Glu Pro Leu Met Glu Gln Val Gly Thr Glu Glu Phe Ile Lys Arg
       130                 135                 140
   Phe Gly Asp Gly Ala Ser Arg Val Val Leu Ser Leu Pro Phe Ala Glu
   145                 150                 155                 160
   Gly Ser Ser Ser Val Glu Tyr Ile Asn Asn Trp Glu Gln Ala Lys Ala
                   165                 170                 175
   Leu Ser Val Glu Leu Glu Ile Asn Phe Glu Thr Arg Gly Lys Arg Gly
               180                 185                 190
   Gln Asp Ala Met Tyr Glu Tyr Met Ala Gln Ala Cys Ala Gly Asn Arg
           195                 200                 205
   Val Arg Arg Ser Val Gly Ser Ser Leu Ser Cys Ile Asn Leu Asp Trp
       210                 215                 220
   Asp Val Ile Arg Asp Lys Thr Lys Thr Lys Ile Glu Ser Leu Lys Glu
   225                 230                 235                 240
   His Gly Pro Ile Lys Asn Lys Met Ser Glu Ser Pro Asn Lys Thr Val
                   245                 250                 255
   Ser Glu Glu Lys Ala Lys Gln Tyr Leu Glu Glu Phe His Gln Thr Ala
               260                 265                 270
   Leu Glu His Pro Glu Leu Ser Glu Leu Lys Thr Val Thr Gly Thr Asn
           275                 280                 285
   Pro Val Phe Ala Gly Ala Asn Tyr Ala Ala Trp Ala Val Asn Val Ala
       290                 295                 300
   Gln Val Ile Asp Ser Glu Thr Ala Asp Asn Leu Glu Lys Thr Thr Ala
   305                 310                 315                 320
   Ala Leu Ser Ile Leu Pro Gly Ile Gly Ser Val Met Gly Ile Ala Asp
                   325                 330                 335
   Gly Ala Val His His Asn Thr Glu Glu Ile Val Ala Gln Ser Ile Ala
               340                 345                 350
   Leu Ser Ser Leu Met Val Ala Gln Ala Ile Pro Leu Val Gly Glu Leu
           355                 360                 365
   Val Asp Ile Gly Phe Ala Ala Tyr Asn Phe Val Glu Ser Ile Ile Asn
       370                 375                 380
   Leu Phe Gln Val Val His Asn Ser Tyr Asn Arg Pro Ala Tyr Ser Pro
   385                 390                 395                 400
   Gly His Lys Thr Gln Pro Phe Leu His Asp Gly Tyr Ala Val Ser Trp
                   405                 410                 415
   Asn Thr Val Glu Asp Ser Ile Ile Arg Thr Gly Phe Gln Gly Glu Ser
               420                 425                 430
   Gly His Asp Ile Lys Ile Thr Ala Glu Asn Thr Pro Leu Pro Ile Ala
           435                 440                 445
   Gly Val Leu Leu Pro Thr Ile Pro Gly Lys Leu Asp Val Asn Lys Ser
       450                 455                 460
   Lys Thr His Ile Ser Val Asn Gly Arg Lys Ile Arg Met Arg Cys Arg
   465                 470                 475                 480
   Ala Ile Asp Gly Asp Val Thr Phe Cys Arg Pro Lys Ser Pro Val Tyr
                   485                 490                 495
   Val Gly Asn Gly Val His Ala Asn Leu His Val Ala Phe His Arg Ser
               500                 505                 510
   Ser Ser Glu Lys Ile His Ser Asn Glu Ile Ser Ser Asp Ser Ile Gly
           515                 520                 525
   Val Leu Gly Tyr Gln Lys Thr Val Asp His Thr Lys Val Asn Ser Lys
       530                 535                 540
   Leu Ser Leu Phe Phe Glu Ile Lys Ser Gly Ser His His His His His
   545                 550                 555                 560
   His
<210> 4
   <211> 562
   <212> PRT
   <213> Artificial Sequence
<220>
   <223> Signal peptide containing Crm197 amino acid sequence expressed from p933
<400> 4
   Met Lys Lys Ile Trp Leu Ala Leu Ala Gly Leu Val Leu Ala Phe Ser
   1               5                   10                  15
   Ala Met Ala Gly Ala Asp Asp Val Val Asp Ser Ser Lys Ser Phe Val
               20                  25                  30
   Met Glu Asn Phe Ser Ser Tyr His Gly Thr Lys Pro Gly Tyr Val Asp
           35                  40                  45
   Ser Ile Gln Lys Gly Ile Gln Lys Pro Lys Ser Gly Thr Gln Gly Asn
       50                  55                  60
   Tyr Asp Asp Asp Trp Lys Glu Phe Tyr Ser Thr Asp Asn Lys Tyr Asp
   65                  70                  75                  80
   Ala Ala Gly Tyr Ser Val Asp Asn Glu Asn Pro Leu Ser Gly Lys Ala
                   85                  90                  95
   Gly Gly Val Val Lys Val Thr Tyr Pro Gly Leu Thr Lys Val Leu Ala
               100                 105                 110
   Leu Lys Val Asp Asn Ala Glu Thr Ile Lys Lys Glu Leu Gly Leu Ser
           115                 120                 125
   Leu Thr Glu Pro Leu Met Glu Gln Val Gly Thr Glu Glu Phe Ile Lys
       130                 135                 140
   Arg Phe Gly Asp Gly Ala Ser Arg Val Val Leu Ser Leu Pro Phe Ala
   145                 150                 155                 160
   Glu Gly Ser Ser Ser Val Glu Tyr Ile Asn Asn Trp Glu Gln Ala Lys
                   165                 170                 175
   Ala Leu Ser Val Glu Leu Glu Ile Asn Phe Glu Thr Arg Gly Lys Arg
               180                 185                 190
   Gly Gln Asp Ala Met Tyr Glu Tyr Met Ala Gln Ala Cys Ala Gly Asn
           195                 200                 205
   Arg Val Arg Arg Ser Val Gly Ser Ser Leu Ser Cys Ile Asn Leu Asp
       210                 215                 220
   Trp Asp Val Ile Arg Asp Lys Thr Lys Thr Lys Ile Glu Ser Leu Lys
   225                 230                 235                 240
   Glu His Gly Pro Ile Lys Asn Lys Met Ser Glu Ser Pro Asn Lys Thr
                   245                 250                 255
   Val Ser Glu Glu Lys Ala Lys Gln Tyr Leu Glu Glu Phe His Gln Thr
               260                 265                 270
   Ala Leu Glu His Pro Glu Leu Ser Glu Leu Lys Thr Val Thr Gly Thr
           275                 280                 285
   Asn Pro Val Phe Ala Gly Ala Asn Tyr Ala Ala Trp Ala Val Asn Val
       290                 295                 300
   Ala Gln Val Ile Asp Ser Glu Thr Ala Asp Asn Leu Glu Lys Thr Thr
   305                 310                 315                 320
   Ala Ala Leu Ser Ile Leu Pro Gly Ile Gly Ser Val Met Gly Ile Ala
                   325                 330                 335
   Asp Gly Ala Val His His Asn Thr Glu Glu Ile Val Ala Gln Ser Ile
               340                 345                 350
   Ala Leu Ser Ser Leu Met Val Ala Gln Ala Ile Pro Leu Val Gly Glu
           355                 360                 365
   Leu Val Asp Ile Gly Phe Ala Ala Tyr Asn Phe Val Glu Ser Ile Ile
       370                 375                 380
   Asn Leu Phe Gln Val Val His Asn Ser Tyr Asn Arg Pro Ala Tyr Ser
   385                 390                 395                 400
   Pro Gly His Lys Thr Gln Pro Phe Leu His Asp Gly Tyr Ala Val Ser
                   405                 410                 415
   Trp Asn Thr Val Glu Asp Ser Ile Ile Arg Thr Gly Phe Gln Gly Glu
               420                 425                 430
   Ser Gly His Asp Ile Lys Ile Thr Ala Glu Asn Thr Pro Leu Pro Ile
           435                 440                 445
   Ala Gly Val Leu Leu Pro Thr Ile Pro Gly Lys Leu Asp Val Asn Lys
       450                 455                 460
   Ser Lys Thr His Ile Ser Val Asn Gly Arg Lys Ile Arg Met Arg Cys
   465                 470                 475                 480
   Arg Ala Ile Asp Gly Asp Val Thr Phe Cys Arg Pro Lys Ser Pro Val
                   485                 490                 495
   Tyr Val Gly Asn Gly Val His Ala Asn Leu His Val Ala Phe His Arg
               500                 505                 510
   Ser Ser Ser Glu Lys Ile His Ser Asn Glu Ile Ser Ser Asp Ser Ile
           515                 520                 525
   Gly Val Leu Gly Tyr Gln Lys Thr Val Asp His Thr Lys Val Asn Ser
       530                 535                 540
   Lys Leu Ser Leu Phe Phe Glu Ile Lys Ser Gly Ser His His His His
   545                 550                 555                 560
   His His
<210> 5
   <211> 561
   <212> PRT
   <213> Artificial Sequence
<220>
   <223> translated protein sequence of expression plasmid p722
<400> 5
   Met Lys Lys Ile Trp Leu Ala Leu Ala Gly Leu Val Leu Ala Phe Ser
   1               5                   10                  15
   Ala Met Gly Ala Asp Asp Val Val Asp Ser Ser Lys Ser Phe Val Met
               20                  25                  30
   Glu Asn Phe Ser Ser Tyr His Gly Thr Lys Pro Gly Tyr Val Asp Ser
           35                  40                  45
   Ile Gln Lys Gly Ile Gln Lys Pro Lys Ser Gly Thr Gln Gly Asn Tyr
       50                  55                  60
   Asp Asp Asp Trp Lys Glu Phe Tyr Ser Thr Asp Asn Lys Tyr Asp Ala
   65                  70                  75                  80
   Ala Gly Tyr Ser Val Asp Asn Glu Asn Pro Leu Ser Gly Lys Ala Gly
                   85                  90                  95
   Gly Val Val Lys Val Thr Tyr Pro Gly Leu Thr Lys Val Leu Ala Leu
               100                 105                 110
   Lys Val Asp Asn Ala Glu Thr Ile Lys Lys Glu Leu Gly Leu Ser Leu
           115                 120                 125
   Thr Glu Pro Leu Met Glu Gln Val Gly Thr Glu Glu Phe Ile Lys Arg
       130                 135                 140
   Phe Gly Asp Gly Ala Ser Arg Val Val Leu Ser Leu Pro Phe Ala Glu
   145                 150                 155                 160
   Gly Ser Ser Ser Val Glu Tyr Ile Asn Asn Trp Glu Gln Ala Lys Ala
                   165                 170                 175
   Leu Ser Val Glu Leu Glu Ile Asn Phe Glu Thr Arg Gly Lys Arg Gly
               180                 185                 190
   Gln Asp Ala Met Tyr Glu Tyr Met Ala Gln Ala Cys Ala Gly Asn Arg
   195 200 205
   Val Arg Arg Ser Val Gly Ser Ser Leu Ser Cys Ile Asn Leu Asp Trp
   210 215 220
   Asp Val Ile Arg Asp Lys Thr Lys Thr Lys Ile Glu Ser Leu Lys Glu
   225 230 235 240
   His Gly Pro Ile Lys Asn Lys Met Ser Glu Ser Pro Asn Lys Thr Val
   245 250 255
   Ser Glu Glu Lys Ala Lys Gln Tyr Leu Glu Glu Phe His Gln Thr Ala
   260 265 270
   Leu Glu His Pro Glu Leu Ser Glu Leu Lys Thr Val Thr Gly Thr Asn
   275 280 285
   Pro Val Phe Ala Gly Ala Asn Tyr Ala Ala Trp Ala Val Asn Val Ala
   290 295 300
   Gln Val Ile Asp Ser Glu Thr Ala Asp Asn Leu Glu Lys Thr Thr Ala
   305 310 315 320
   Ala Leu Ser Ile Leu Pro Gly Ile Gly Ser Val Met Gly Ile Ala Asp
   325 330 335
   Gly Ala Val His His Asn Thr Glu Glu Ile Val Ala Gln Ser Ile Ala
   340 345 350
   Leu Ser Ser Leu Met Val Ala Gln Ala Ile Pro Leu Val Gly Glu Leu
   355 360 365
   Val Asp Ile Gly Phe Ala Ala Tyr Asn Phe Val Glu Ser Ile Ile Asn
   370 375 380
   Leu Phe Gln Val Val His Asn Ser Tyr Asn Arg Pro Ala Tyr Ser Pro
   385 390 395 400
   Gly His Lys Thr Gln Pro Phe Leu His Asp Gly Tyr Ala Val Ser Trp
   405 410 415
   Asn Thr Val Glu Asp Ser Ile Ile Arg Thr Gly Phe Gln Gly Glu Ser
   420 425 430
   Gly His Asp Ile Lys Ile Thr Ala Glu Asn Thr Pro Leu Pro Ile Ala
   435 440 445
   Gly Val Leu Leu Pro Thr Ile Pro Gly Lys Leu Asp Val Asn Lys Ser
   450 455 460
   Lys Thr His Ile Ser Val Asn Gly Arg Lys Ile Arg Met Arg Cys Arg
   465 470 475 480
   Ala Ile Asp Gly Asp Val Thr Phe Cys Arg Pro Lys Ser Pro Val Tyr
   485 490 495
   Val Gly Asn Gly Val His Ala Asn Leu His Val Ala Phe His Arg Ser
   500 505 510
   Ser Ser Glu Lys Ile His Ser Asn Glu Ile Ser Ser Asp Ser Ile Gly
   515 520 525
   Val Leu Gly Tyr Gln Lys Thr Val Asp His Thr Lys Val Asn Ser Lys
   530 535 540
   Leu Ser Leu Phe Phe Glu Ile Lys Ser Gly Ser His His His His His
   545 550 555 560
   His
<210> 6
   <211> 535
   <212> PRT
   <213> Corynebacterium diphtheriae
<220>
   <223> secreted mature CRM197 protein
<400> 6
   Gly Ala Asp Asp Val Val Asp Ser Ser Lys Ser Phe Val Met Glu Asn
   1 5 10 15
   Phe Ser Ser Tyr His Gly Thr Lys Pro Gly Tyr Val Asp Ser Ile Gln
   20 25 30
   Lys Gly Ile Gln Lys Pro Lys Ser Gly Thr Gln Gly Asn Tyr Asp Asp
   35 40 45
   Asp Trp Lys Glu Phe Tyr Ser Thr Asp Asn Lys Tyr Asp Ala Ala Gly
   50 55 60
   Tyr Ser Val Asp Asn Glu Asn Pro Leu Ser Gly Lys Ala Gly Gly Val
   65 70 75 80
   Val Lys Val Thr Tyr Pro Gly Leu Thr Lys Val Leu Ala Leu Lys Val
   85 90 95
   Asp Asn Ala Glu Thr Ile Lys Lys Glu Leu Gly Leu Ser Leu Thr Glu
   100 105 110
   Pro Leu Met Glu Gln Val Gly Thr Glu Glu Phe Ile Lys Arg Phe Gly
   115 120 125
   Asp Gly Ala Ser Arg Val Val Leu Ser Leu Pro Phe Ala Glu Gly Ser
       130                 135                 140
   Ser Ser Val Glu Tyr Ile Asn Asn Trp Glu Gln Ala Lys Ala Leu Ser
   145                 150                 155                 160
   Val Glu Leu Glu Ile Asn Phe Glu Thr Arg Gly Lys Arg Gly Gln Asp
                   165                 170                 175
   Ala Met Tyr Glu Tyr Met Ala Gln Ala Cys Ala Gly Asn Arg Val Arg
               180                 185                 190
   Arg Ser Val Gly Ser Ser Leu Ser Cys Ile Asn Leu Asp Trp Asp Val
           195                 200                 205
   Ile Arg Asp Lys Thr Lys Thr Lys Ile Glu Ser Leu Lys Glu His Gly
       210                 215                 220
   Pro Ile Lys Asn Lys Met Ser Glu Ser Pro Asn Lys Thr Val Ser Glu
   225                 230                 235                 240
   Glu Lys Ala Lys Gln Tyr Leu Glu Glu Phe His Gln Thr Ala Leu Glu
                   245                 250                 255
   His Pro Glu Leu Ser Glu Leu Lys Thr Val Thr Gly Thr Asn Pro Val
               260                 265                 270
   Phe Ala Gly Ala Asn Tyr Ala Ala Trp Ala Val Asn Val Ala Gln Val
           275                 280                 285
   Ile Asp Ser Glu Thr Ala Asp Asn Leu Glu Lys Thr Thr Ala Ala Leu
       290                 295                 300
   Ser Ile Leu Pro Gly Ile Gly Ser Val Met Gly Ile Ala Asp Gly Ala
   305                 310                 315                 320
   Val His His Asn Thr Glu Glu Ile Val Ala Gln Ser Ile Ala Leu Ser
                   325                 330                 335
   Ser Leu Met Val Ala Gln Ala Ile Pro Leu Val Gly Glu Leu Val Asp
               340                 345                 350
   Ile Gly Phe Ala Ala Tyr Asn Phe Val Glu Ser Ile Ile Asn Leu Phe
           355                 360                 365
   Gln Val Val His Asn Ser Tyr Asn Arg Pro Ala Tyr Ser Pro Gly His
       370                 375                 380
   Lys Thr Gln Pro Phe Leu His Asp Gly Tyr Ala Val Ser Trp Asn Thr
   385                 390                 395                 400
   Val Glu Asp Ser Ile Ile Arg Thr Gly Phe Gln Gly Glu Ser Gly His
                   405                 410                 415
   Asp Ile Lys Ile Thr Ala Glu Asn Thr Pro Leu Pro Ile Ala Gly Val
               420                 425                 430
   Leu Leu Pro Thr Ile Pro Gly Lys Leu Asp Val Asn Lys Ser Lys Thr
           435                 440                 445
   His Ile Ser Val Asn Gly Arg Lys Ile Arg Met Arg Cys Arg Ala Ile
       450                 455                 460
   Asp Gly Asp Val Thr Phe Cys Arg Pro Lys Ser Pro Val Tyr Val Gly
   465                 470                 475                 480
   Asn Gly Val His Ala Asn Leu His Val Ala Phe His Arg Ser Ser Ser
                   485                 490                 495
   Glu Lys Ile His Ser Asn Glu Ile Ser Ser Asp Ser Ile Gly Val Leu
               500                 505                 510
   Gly Tyr Gln Lys Thr Val Asp His Thr Lys Val Asn Ser Lys Leu Ser
           515                 520                 525
   Leu Phe Phe Glu Ile Lys Ser
       530                 535

## Description

### 1 INTRODUCTION

The present invention provides novel methods of producing diphtheria toxin. In particular, the present invention provides novel methods of producing nontoxic forms of diphtheria toxin, e.g., CRM197. The present invention also provides novel compositions comprising diphtheria toxin or nontoxic forms of diphtheria toxin, e.g., CRM197.

### 2 BACKGROUND

The CRM197 protein is a safe and effective T-cell dependent carrier for saccharides and is currently being used in many different vaccine formulations called conjugate vaccines. Diphtheria toxin is a protein exotoxin produced by the bacterium *Corynebacterium diphtheriae* upon infection with the phage β197. Both, Diphtheria toxin ("DT") and CRM197 are components of many vaccines, like for example against *Bordatella pertussis, Clostridium tetani, C. diphtheriae,* hepatitis B virus, and *Haemophilus influenza* type B (WO 9324148, WO 9700697, WO 02055105). In addition there has been a growing interest in CRM197 because of its potential antitumor activity relating to its capacity to bind the soluble form of HB-EGF (US 2006/0270600A1).

CRM197 is produced by *C*. *diphtheriae* infected by the non-toxigenic phage β197tox. β197tοx was created by nitrosoguanidine mutagenesis of the toxigenic corynephage β (Uchida, T. et al. 1971, Nature New Biology 233:8-11). The CRM197 protein is a nontoxic form of diphtheria toxin but is immunologically indistinguishable from the diphtheria toxin. DT has a mass of 58.350 kDa (CRM197 = 58.415 kDa) and consists of the N-terminal A and the C-terminal B domains (21 and 37 kDa) which are linked by a disulfide bridge connecting Cys186 and Cys201. The A fragment is toxic after being released from its disulfide-bonded partner, the B fragment. Nicking of the holotoxin by mild proteolysis at the connecting peptide at positions 191-3 is a prerequisite for the A fragment activation. The B fragment has no apparent enzymatic activity but is required for toxicity, probably due to targeting the holotoxin to the target cell membranes (Broker M, Costantino P, De Tora L, McIntosh ED, Rappuoli R: Biochemical and biological characteristics of cross-reacting material 197 (CRM197), a non-toxic mutant of diphtheria toxin: use as a conjugation protein in vaccines and other potential clinical applications. Biologicals, 2011, 39(4):195-204.)

Infected *C*. *diphtheriae* cultures secrete the CRM197 protein across the cytoplasmic membrane out of the cell into the culture medium. The CRM197 protein has about the same molecular weight as the diphtheria toxin but differs therefrom by a single base change (guanine to adenine) in the structural gene. This single base change causes an amino acid substitution (glutamic acid for glycine, G52E) in the mature protein and eliminates the toxic properties of diphtheria toxin (Giannini G, Rappuoli R, Ratti G: The amino-acid sequence of two non-toxic mutants of diphtheria toxin: CRM45 and CRM197. Nucleic Acids Res 1984, 12(10):4063-4069).

Methods of preparing DT and CRM197 are described in US 4709017, US 5843711, US 5601827, and US 5917017. There are currently three different systems used for industrial preparation of CRM197. Two systems are based on the use of phage infected *C*. *diphtheriae* cells. The most recent development constitutes a recombinant expression system in *Pseudomonas fluorescens.* The method employs a secretion approach to the periplasm in a genetically optimized *P. fluorescens* strain using a CRM197 gene equipped with a signal peptide for secretion into the periplasm (US20110287443).

For example, diphtheria toxin is isolated from cultures of *C*. *diphtheriae* strain C7 (B197) and/or *C*. *diphtheriae* strain C7 (B197) pPx350 grown in a casamino acids and yeast extract-based medium under aerobic conditions. Adjustment of media components were shown to improve yields (US 4925792, WO 2006 100108). CRM197 or DT are harvested from the supernatant of the culture, and concentrated by ultrafiltration. Ammonium sulfate precipitation is a first, and anionic exchange chromatography a second purification step.

However, production of significant quantities of the CRM197 protein for use in vaccines has been hindered due to low protein abundance (WO 2006 100108).

Techniques have been developed to bolster the production of CRM proteins using double lysogens (Isolation and characterization of *C*. *diphtheriae* nontandem double lysogens hyperproducing CRM197. R Rappuoli, Appl. Environ. Microbiol. September 1983 46:560-564; U.S. Pat. No. 4,925,792 issued to R. Rappuoli; and Integration of corynebacteriophages beta tox+, omega tox+, and gamma tox- into two attachment sites on the *C. diphtheriae* chromosome. R Rappuoli, J L Michel, and J R Murphy; J. Bacteriol. March 1983 153:1202-1210) of the nontoxigenic corynephage β197. Rappuoli reports yields of CRM197 from double and triple lysogens up to three fold higher than from the single lysogens. The production levels of CRM197 by single lysogens are adequate but economically unsatisfactory for the production of vaccines which utilize CRM197 protein. It is important to note that the construction of double and triple lysogenic strains in order to increase expression efficiency in *C*. *diphtheria e* is a long process which requires a laborious screening phase.

Plasmids were developed for recombinant expression of CRM197 in *C. diphtheriae* (US Patent US patent 5614382, 1995/5614382 _1997). This makes it possible to increase the number of copies of the gene (up to 5-10 per cell) without having to select pluri-lysogenic bacterial strains.

As in the case of the *Corynebacterium* strains infected by the phage β197tοx, CRM197 is expressed in special culture media with a low ferrous content. Despite a reduction in the amount of time required for the genetic handling of the bacterial strain, the output of CRM197 does not increase dramatically by comparison with the use of double lysogenes.

Alternative expression host cells for DT included a *Salmonella typhi* vaccine strain cvd 908-htra (Orr N, Galen JE, Levine MM: Expression and immunogenicity of a mutant diphtheria toxin molecule, CRM197, and its fragments in S. typhi vaccine strain CVD 908-htrA. Infect Immun 1999, 67(8):4290-4294). *Salmonella* is a Gram negative bacterium and similar expression host as *E. coli.* Expression levels from various constructs (with, without signal peptide) in cvd 908-htra were low and solubility and immunogenicity were poor. Utilizing the alternative, non-Sec dependent translocation system of the hemolysin operon improved expression of soluble DT, but levels were still low.

Reports for production of CRM197 in *E. coli* show low yields of soluble CRM197 and formation of insoluble product in inclusion bodies. Truncation approaches have been used in an attempt to enhance expression to higher levels. (Bishai WR, Miyanohara A, Murphy JR: Cloning and expression in E. coli of three fragments of diphtheria toxin truncated within fragment B. Journal of Bacteriology 1987, 169(4):1554-1563)

A single strand expression plasmid for CRM197 containing the mutated diphtheria toxin gene encoding CRM197 was used for expression in *E. coli* (Bishai WR, Rappuoli R, Murphy JR: High-level expression of a proteolytically sensitive diphtheria toxin fragment in Escherichia coli. Journal of Bacteriology 1987, 169(11):5140-5151; Bishai 1987). In this publication, transcription of CRM197 was controlled by the endogenous and constitutive Ptox promotor. In addition, DT C-terminally fused to the alpha melanocyte stimulating hormone ("ABM508") was expressed by the heat inducible P_{Lambda} promoter or the P*_{tac}* promoter for expression.

Bishai 1987 speculated that jamming of the secretion apparatus due to high level protein induction caused a growth stop after induction of expression of periplasmic DT/CRM197 variants. This can be a general problem in periplasmic protein expression that has been observed and resulted in low volumetric yields of protein previously (Benson SA, Hall MN, Silhavy TJ: Genetic analysis of protein export in E. coli K12. Annual Review of Biochemistry 1985, 54:101-134). Jamming of the secretion apparatus and formation of insoluble protein suggested an inability of the *E. coli* cells to provide a productive translocation and folding environment for CRM197 biogenesis.

As a consequence, Bishai 1987 reasoned that cytoplasmic expression would avoid the translocon jamming. Thus, Bishai 1987 removed the signal peptide for directing the expression into the cytoplasm. Only at low temperatures and when cytoplasmic proteases were deleted, did the cytoplasmic expression constructs yield soluble product. Production was inefficient and lead to aggregates at elevated temperatures, and when proteases were present.

Bishai 1987 failed to show production of high levels of soluble protein CRM197 fusion protein, *i.e.,* with signal peptide for periplasmic targeting. In a Coomassie stained SDS polyacrylamide gel, extracts containing the ABM508 expression construct showed an intense protein band corresponding to ABM508, whereas cells expressing CRM197 expressed from the natural promoter with the wild type signal peptide do not show an obvious band for CRM197 at the expected size of 58 kDa.

Thus, periplasmic expression was not considered an efficient production strategy and to date there is no efficient *E. coli* periplasmic expression system established for production of soluble and correctly folded CRM197 or DT.

A production system which is based on the cytoplasmic expression of insoluble CRM197 in inclusion bodies followed by solubilization, purification and refolding of the protein is provided by the first *E. coli* based expression system for CRM197 (WO2010 150230). Wild-type CRM197 without additional amino acids can only be obtained with this system when an additional proteolysis step is applied.

Signal peptides induce protein secretion to the periplasm and have various effects on protein biogenesis. (Powers T, Walter P: Co-translational protein targeting catalyzed by the E. coli signal recognition particle and its receptor. The EMBO Journal 1997, 16(16):4880-4886.) (Schierle CF, Berkmen M, Huber D, Kumamoto C, Boyd D, Beckwith J: The DsbA signal sequence directs efficient, cotranslational export of passenger proteins to the E. coli periplasm via the signal recognition particle pathway. Journal of Bacteriology 2003, 185(19):5706-5713). The expression and purification of CRM197 has been disclosed in WO 11/42516 and Alessandra Stefan et al Journal of Biotechnology 156; 245-252 (2011).

### 3 SUMMARY

Provided herein is a method of producing CRM197 wherein the method comprises culturing an *E. coli* cell comprising a nucleic acid encoding CRM197 wherein CRM197 is fused to a heterologous signal peptide that targets CRM197 to the periplasm of the *E. coli* cell, wherein the nucleic acid encodes a cleavage site between the signal peptide that targets CRM197 to the periplasm and the CRM197 protein wherein the cleavage site comprises the amino acids sequence aa1-aa2-aa3-(cleavage site)-aa4-aa5-aa6-aa7-aa8, wherein aa1 is Ala, Ser, Gly, Cys, Thr or Gln; aa2 is selected from any natural amino acid; aa3 is selected from any natural amino acid except Phe, His, Tyr, Trp, Asp, Glu, Lys, Arg, Asn and Gln; aa4 to 8 is selected from ala-asp-asp-val and met-gly-ala-asp; or wherein the cleavage site comprises the amino acid sequence aa1-aa2-aa3-(cleavage site)-aa4-aa5-aa6-aa7-aa8, wherein aa4 to 8 is selected from ala-asp-asp-val and met-gly-ala-asp; and wherein the first 70 aa of the first open reading frame results in a Y score when analysed by SignalP4.0 Server of more than 0.72. The method is to produce soluble, folded, full length CRM197 at high yields (e.g., at least 0.5 mg/1) in *E. coli* expression strains. In particular, a signal peptide is used to direct secretion of the protein into the periplasmic space.

Provided herein are methods for producing CRM197 wherein the method comprises culturing an *E. coli* cell comprising a nucleic acid encoding CRM197 wherein CRM197 is fused to a heterologous signal peptide that targets CRM197 to the periplasm of the *E. coli* cell. In more specific embodiments, the wild type signal peptide of CRM197 has been deleted. In even more specific embodiments, the wild type signal peptide of CRM197 has been replaced by the heterologous signal peptide. The heterologous signal peptide can be selected from the group consisting of the signal peptide from *E. coli* heat-labile enterotoxin, *E. coli* outer membrane porin (OmpA), *E. coli* maltose binding protein (MalE), *E. carotovorans* pectate lyase (PelB), and *Bacillus* sp. endoxylanase (XynA). CRM197 can be produced at a concentration of at least 5, 10, 25, 50, 75, 100, 125, 125, 150, 175, 200, 225, 250, 300, 400, 500, 600, 700, 800, 900, or at least 1000 mg protein per liter culture medium. At least 50% of the produced protein is properly folded as determined by circular dichroism. At least 10%, 20%, 30%, 40%, 50%, 60%, 70%, 80%, 90%, 95%, 98%, 99%, 99.5%, 99.8%, or at least 99.9% of the produced protein is properly folded. At least 50% of the produced protein is not present in aggregates. At least 10%, 20%, 30%, 40%, 50%, 60%, 70%, 80%, 90%, 95%, 98%, 99%, 99.5%, 99.8%, or at least 99.9% of the produced protein is not present in aggregates. Aat least 50% of the produced protein is soluble. At least 10%, 20%, 30%, 40%, 50%, 60%, 70%, 80%, 90%, 95%, 98%, 99%, 99.5%, 99.8%, or at least 99.9% of the produced protein is soluble.

In certain embodiments, the heterologous nucleotide sequence encodes a cleavage site between the signal peptide that targets CRM197 to the periplasm and the CRM197 protein wherein the cleavage site comprises the amino acid sequence aa1-aa2-aa3-(cleavage site)-aa4-aa5-aa6-aa7-aa8, wherein
aa1 is selected from Ala, Ser, Gly, Cys, Thr, and Gln;
aa2 is selected from any natural amino acid;
aa3 is selected from any natural amino acid except Phe, His, Tyr, Trp, Asp, Glu, Lys, Arg Asn, and Gln;
aa4 to 8 is selected from ala-asp-asp-val and met-gly-ala-asp;
or wherein the cleavage site comprises the amino acid sequence aa1-aa2-aa3-(cleavage site)-aa4-aa5-aa6-aa7-aa8, wherein aa4 to 8 is selected from ala-asp-asp-val and met-gly-ala-asp; and wherein the first 70 aa of the open reading frame results in a Y score when analyzed by SignalP 4.0 Server of more than 0.72.

In certain specific embodiments, the heterologous nucleotide sequence encodes the protein of SEQ ID NO: 1 or 2. The heterologous nucleotide sequence can be operatively linked to a promoter selected from the group consisting of the 1-arabinose inducible araBAD promoter (PBAD), the lac promoter, the 1-rhamnose inducible rhaP BAD promoter, the T7 RNA polymerase promoter, the trc and tac promoter, the lambda phage promoter p L , and the anhydrotetracycline-inducible tetA promoter/operator.

In certain embodiments, the nucleic acid encoding CRM197 is inserted in a high copy expression plasmid. The high copy expression plasmid can be pEC415, pBR322, pBAD, pET series, pUC series, pACT3, pEXT22, pEXT20, pBLUESCRIPT series, pGEM series.

In certain embodiments, the expression of CRM197 can be induced at a culture density of OD600 >0.3. Specifically, the expression of CRM197 can be induced at a culture density of OD600 >0.5, >1, or >1.5.

CRM197 can be expressed at a temperature of 37°C. CRM197 can be expressed at a temperature of 20, 25, 30, 32, or 35°C.

In certain embodiments, at least 50% of CRM197 protein that has been produced in accordance with the methods provided herein have an N-terminus of ADDV, GADDV, or MGADDV. More specifically, at least 60%, 70%, 80%, 90%, 95%, 98%, 99%, at least 99.5% or 100% of the expressed CRM197 have an N-terminus of ADDV, GADDV, or MGADDV. In certain embodiments, at least 50% of the expressed CRM197 have a disulfide bond between Cys186 and Cys201. At least 60%, 70%, 80%, 90%, 95%, 98%, 99%, at least 99.5% or 100% of the expressed CRM197 have a disulfide bond between Cys186 and Cys201.

### 3.1 Terminology

DT-diphtheria toxin
CRM197-Cross reactive material 197, DT with a mutation of glycine 52 to glutamate, G52E
AMB508-fusion protein consisting of alpha melanocyte-stimulating hormone sequence fused to the CRM197 sequence
β197tox-non-toxigenic corynephage 197
preprotein-protein including the signal peptide
mature protein, processed protein- protein after signal peptide cleavage
IPTG-Isopropyl-β-D-thiogalactopyranosid, inducer for lac, trc, and related promoters, to be added to the growth media at induction time
ParaBAD-promoter of the araBAD operon, inducible by the addition of L-arabinose to the growth media

### 4 BRIEF DESCRIPTION OF THE DRAWINGS

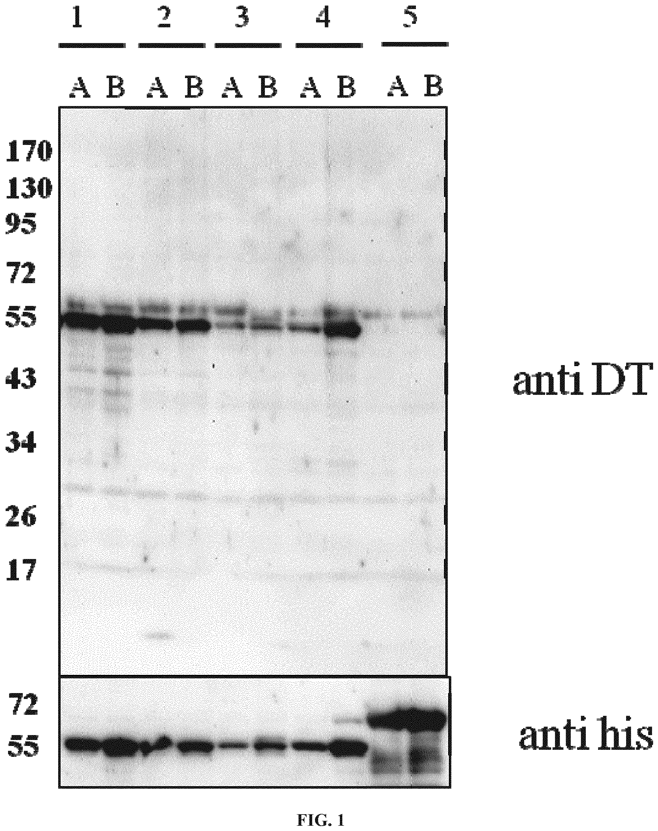

Fig. 1 Expression of CRM197 in *E. coli.* Different expression plasmids (1, 2, 3, 4, 5 indicating p932, p934, p722, a cytoplasmic CRM197 variant, and p150 (see, Table 2)) were tested for CRM197 expression in *E. coli* BL21 cells (lanes indicated by A) or W3110 (B). Total cell extracts normalized to OD600 after 2 hours of induction were prepared and analyzed. The top panel shows a Western blot using anti DT antiserum, bottom panel was detected using anti his tag antiserum.

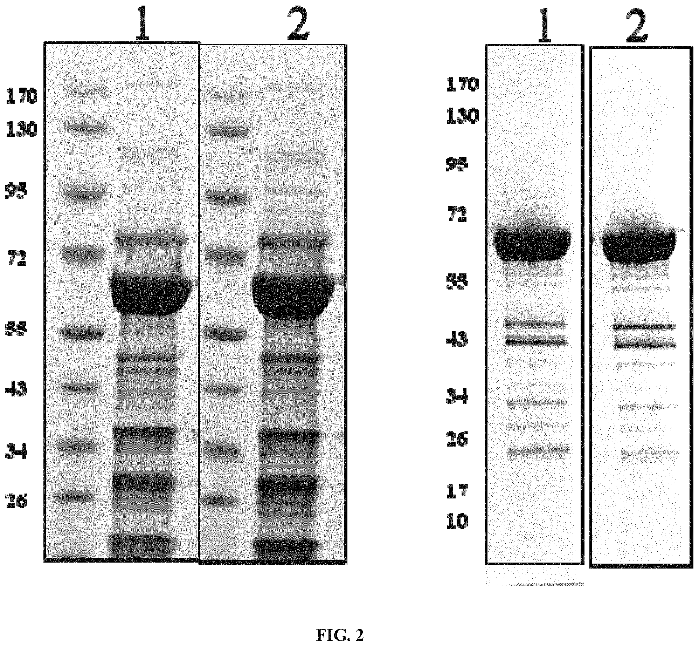

Fig. 2. Purification of soluble CRM197 from *E. coli.* Two different strains containing expression plasmids (1, 2, indicating p932, p933) in BL21 were grown as described in the text. Elution fractions were separated by SDS PAGE and stained by Coomassie blue (left panel) and immunodetected after electrotransfer to nitrocellulose membranes using anti DT antiserum.

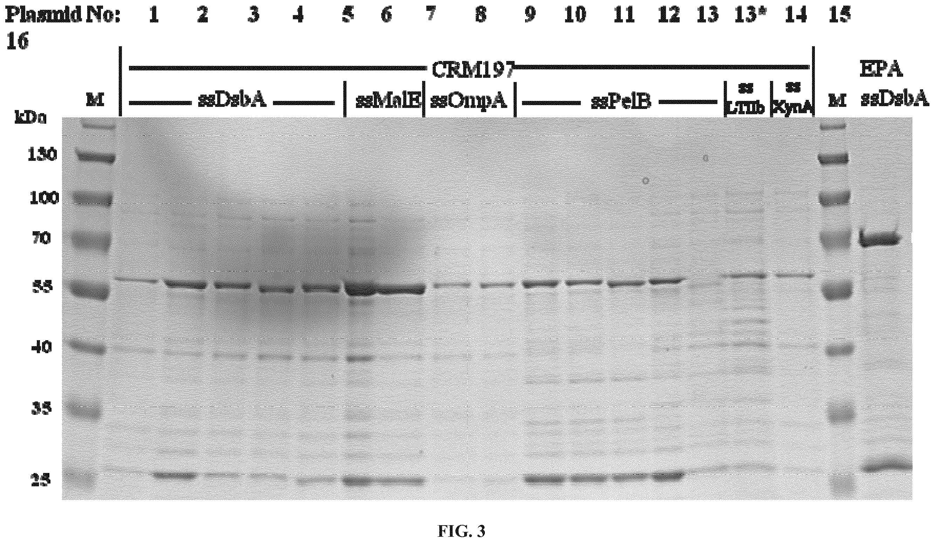

Fig. 3. Periplasmic expression levels of CRM197 fused to different N-terminal signal peptides in comparison to a well-secreted reference protein EPA-6H, recombinant, genetically detoxified *Pseudomonas aeruginosa* exotoxin A (EPA), (Ihssen J, Kowarik M, Dilettoso S, Tanner C, Wacker M, Thony-Meyer L: Production of glycoprotein vaccines in E. coli. Microbial cell factories 2010, 9:61). Coomassie stained SDS-PAGE gel with periplasmic extracts of *E. coli* strains harboring plasmids 1 to 16 as described in Table 2. Cells were grown in shake flasks at the optimal expression temperature in TB medium and induced at an OD₆₀₀ of 0.4-0.6 by the addition of 4 g/l L-arabinose. OD equivalent samples for extraction of soluble periplasmic proteins (sucrose-lysozyme method) were taken 4 h after induction, with the exception of 13*: sample taken before induction. M: protein marker mix, Thermo-Scientific prestained protein ladder #26616, 3 µL (concentration of individual proteins 0.1-0.2 mg / ml).

### 5 DETAILED DESCRIPTION

Provided herein are methods for the expression of diphtheria toxin and nontoxic forms of diphtheria toxin, e.g., CRM197. More specifically, provided herein are methods for the expression of CRM197 and secretion of CRM197 into the periplasmic space of *E. coli* cells in a soluble and correctly folded form.

CRM197 is expressed using a heterologous signal peptide that targets CRM197 in the periplasmic space of the host cell. In certain more specific embodiments, the host cell is *E. coli.* An expression cassette containing the CRM197 gene and a heterologous signal peptide can be constructed using standard molecular biology techniques. Specifically, the wild type signal peptide of CRM197 is deleted and a heterologous signal peptide is introduced instead. On a nucleic acid level, care should be taken that the sequence encoding the signal peptide is cloned in frame with the nucleic acid encoding the remainder of CRM197. In certain specific embodiments, the heterologous signal peptide replaces the wild type signal peptide. In other embodiments, the wild type signal peptide is deleted or functionally inactivated and the heterologous signal peptide is introduced at a different location of the protein. In certain embodiments, a proteolytic cleavage site is introduced between the signal peptide and the remainder of the molecule. The proteolytic cleavage site can be recognized and cleaved in the periplasm of the host cell. In certain more specific embodiments, the signal peptidase is recombinantly expressed in the host cell.

In certain embodiments, the following parameters can affect the expression of the protein of interest. More detailed information on these various aspects are provided in the following sections.

The nucleic acid can encode the mature, secreted CRM197 (SEQ ID NO:6):
i) in a fashion optimized for *E. coli* codon usage.
ii) A heterologous signal sequence can be used for targeting CRM197 to the periplasmic space in *E. coli.* By standard cloning procedures, synthetic DNA sequences encoding a heterologous signal peptide can be fused at the N-terminus of the mature CRM197 gene. Different N-terminal signal peptides such as from *E. coli* heat-labile enterotoxin, *E. coli* outer membrane porin A (OmpA), *E. coli* maltose binding protein (MalE), *E. coli* DsbA, *Erwinia carotovorans* pectate lyase (PelB), or *Bacillus* sp. endoxylanase (XynA), can be used with the methods provided herein. In certain embodiments, a particular heterologous signal peptide has been demonstrated to confer secretion of recombinant proteins into the periplasmic space of *E. coli.*
iii) The signal peptide cleavage site (*i.e.* the sequence between the signal peptide and the secreted protein); For example, signal peptide cleavage prediction programs, such as for example SignalP 4.0 server program (hosted at the website of the Center for Biological Sequence Analysis of the Technical University of Denmark), can be used to design alternative signal peptide cleavage sites. This program predicts i) cleavage site probability, and ii) cleavage site location, i.e. between which amino acids cleavage is most likely to occur. In specific embodiments, signal peptide cleavage sites are designed in a way to result in a CRM197 N terminus as similar as possible to the natural N- terminus.
iv) A suitable high copy number expression plasmid can be used with the methods provided herein.
v) Expression of CRM 197 can be positioned under the control of the high level induction arabinose promoter.
vi) The growth medium;
vii) The expression time, i.e. the point of induction during growth and the time between induction and harvest of the cells;
viii) Amount of inducer;
ix) The expression temperature;

In general, a method provided herein is conducted as follows. First, an expression plasmid as described herein is introduced into a host cell (e.g., *E. coli* or *Salmonella* sp expression strain). The transformation mix can be plated on rich media supplemented with the antibiotic for which the expression plasmid carries a resistance marker. A single colony can be used to inoculate a small culture volume (e.g., 5 ml) consisting of, e.g., TB medium, or a similar rich medium containing glycerol as a carbon source and lacking or supplemented with the suitable antibiotic. The culture can then be incubated between 20-35°C until stationary phase and then diluted to fresh medium of identical or similar composition, pre-warmed at 20-35°C, at a ratio of 1:50 to 1:100. The fresh culture can then be grown to exponential growth phase (OD₆₀₀ of 0.6-1.2) and expression is induced by the addition of the appropriate inducer, which depends on the promoter used in the expression plasmid. Examples of inducers include arabinose or a different chemical of physical condition for high level protein induction. Then, expression is continued. In certain embodiments, expression is continued before harvesting for at least 2, 4, 6, 8, 10, 12, 14, 16, 18, 20, 22, 24, 26, 28, or at least 30 hours.

Samples can be taken at any time. At all-time points the amount of soluble CRM197 formed is evaluated by analysis via SDS-PAGE and Coomassie staining of periplasmic extracts. A comparison to commercial CRM197 of known concentration allows estimating the yield.

In certain embodiments, the following control is used to determine that the expressed CRM197 protein is soluble. Without being bound by theory, such soluble protein is correctly folded. CRM197 detected at around 58 kDa in periplasmic extracts prepared by the sucrose-lysozyme method (Kowarik M, Young NM, Numao S, Schulz BL, Hug I, Callewaert N, Mills DC, Watson DC, Hernandez M, Kelly JF et al: Definition of the bacterial N-glycosylation site consensus sequence. The EMBO journal 2006, 25(9):1957-1966) can be used as a standard for soluble protein.

In certain embodiments, provided herein are methods that result in a concentration of properly folded CRM197 of at least 1 mg/1, 2 mg/l, 3 mg/l, 4 mg/l, 5 mg/l, 6 mg/l, 7 mg/l, 8 mg/l, 9 mg/l, 10 mg/l, 11 mg/l, 12 mg/l, 13 mg/l, 14 mg/l, 15 mg/l, 20 mg/l, 25 mg/l, 50 mg/l, 75 mg/l, or at least 100 mg/l.

In certain embodiments, depending on the signal peptide cleavage site sequence, the N terminus of CRM197 can be ADDV, or MGADDV.

In certain embodiments, at least 50%, 60%, 70%, 75%, 80%, 85%, 90%, 95%, 98%, 99%, or 100% of the resulting CRM197 proteins have a disulfide bond between Cys186 and Cys201 connecting A and B fragments. The presence of this disulfide bond can be demonstrated using a thiol assay (Hansen RE, Ostergaard H, Norgaard P, Winther JR: Quantification of protein thiols and dithiols in the picomolar range using sodium borohydride and 4,4'-dithiodipyridine. Anal Biochem 2007, 363(1):77-82).

In certain embodiments, at least 50%, 60%, 70%, 75%, 80%, 85%, 90%, 95%, 98%, 99%, or 100% of the resulting CRM197 proteins are soluble and are not present in aggregates.

### 5.1 Proteins of Interest

The present invention describes production methods for CRM197. Illustrative protein sequences for CRM197 are provided as SEQ ID NOs: 3 and 4, and the corresponding full length expression plasmid DNA sequences are SEQ ID NOs: 1 and 2.

### 5.2 Periplasmic Targeting

Various secretory pathways from the cytoplasm to the periplasm exist in *E. coli.* These pathways include the Sec pathway, the SRP dependent pathway, and the twin arginine pathway for secretion. The TatABC pore is thought to be responsible for the secretion of folded proteins. Without being bound by theory, the signal peptide can determine which secretory pathway is chosen by the cell. (Driessen AJ, Nouwen N: Protein translocation across the bacterial cytoplasmic membrane. Annual review of biochemistry 2008, 77:643-667.)

In certain embodiments, the heterologous signal peptide for use with the present methods is an amino-terminal hydrophobic signal sequences that is cleaved during the translocation process. In certain embodiments, conditions for a method provided herein are chosen such that the protein does not fold into its stable three-dimensional structure in the cytoplasm of the host cell. Without being bound by theory, folding in the cytoplasm may prevent export. In certain other embodiments, the heterologous signal peptide encodes an arginine signature that targets the protein to the twin arginine pathway for secretion.

In certain embodiments, an unfolded protein may be maintained in an export competent state in several different ways: (i) the heterologous signal peptide can be chosen such that the protein may be translocated across a membrane simultaneously with translation of the protein, thus ensuring that not even its secondary structures are formed in the cytoplasm due to the absence of amino acid polymer; (ii) chaperones or antifolding factors that prevent folding in the cytoplasm (Randall LL, Topping TB, Smith VF, Diamond DL, Hardy SJ: SecB: a chaperone from E. coli. Methods Enzymol 1998, 290:444-459.) can be provided; (iii) the heterologous signal sequences is chosen and/or inserted such that it acts as intrapolypeptide chaperones to prevent rapid folding; and/or (iv) the DT or CRM197 is modified such that it contains features in its final structure (e.g., disulfide bonds) that do not form in the environment of the cytoplasm so that the proteins cannot attain their final folded conformations in the cytoplasm.

### 5.2.1 Signal Peptides

Illustrative heterologous signal peptides that can be used with the methods provided herein are: the *E. coli* DsbA signal sequence, the MalE, OmpA, and PelB signal peptides. Without being bound by theory, the choice of signal peptide can determine the secretion route, e.g., SRP-dependent vs. SecB dependent route to the translocon. The optimal expression conditions may differ for different targeting pathways. There are reports claiming technologies that allow the identification of the targeted secretion pathway (Marrichi M, Camacho L, Russell DG, DeLisa MP: Genetic toggling of alkaline phosphatase folding reveals signal peptides for all major modes of transport across the inner membrane of bacteria. J Biol Chem 2008, 283(50):35223-35235).

The preferred signal peptides are selected from known and predicted, secreted proteins which are efficiently exported to the periplasm of E. *coli* via co-translational pathways. Among others, the signal peptides of *E. coli* heat-labile enterotoxin, *E. coli* outer membrane porin A (OmpA), *E. coli* maltose binding protein (MalE), *E. carotovorans* pectate lyase (PelB), or *Bacillus* sp. endoxylanase (XynA) can be used.

### 5.2.2 Cleavage Sites

Without being bound by theory, signal peptides are cleaved off the preprotein by a signal peptidase, and in *E. coli* there are SPaseI and II. SPaseI is cleaving most soluble and some membrane protein signal peptides, whereas SPaseII cleaves signal peptides from lipoproteins. SPaseI is the signal peptidase responsible in the presented invention. SPaseI usage could be determined (Paetzel M, Karla A, Strynadka NC, Dalbey RE: Signal peptidases. Chemical reviews 2002, 102(12):4549-4580).

Without being bound by theory, cleavage site locations are defined by i) the structure of the characteristic signal peptide organization with a hydrophobic core, a charged N terminus and a hydrophilic C terminus, and ii) by the primary sequence around the cleavage position (often A-X-A) (Heijne G: The distribution of positively charged residues in bacterial inner membrane proteins correlates with the trans-membrane topology. The EMBO journal 1986, 5(11):3021-3027.). Both parameters are well understood and prediction programs have a high accuracy (Petersen TN, Brunak S, von Heijne G, Nielsen H: SignalP 4.0: discriminating signal peptides from transmembrane regions. Nat Methods 2011, 8(10):785-786). The program SignalP 4.0 server provides a cleavage probability based on the sequence of the first 70 amino acids of the pre-protein. In certain embodiments, engineered cleavage sites for use with the methods provided herein have a Y score of at least 0.4, 0.5, 0.6, 0.7, 0.72. 0.75, 0.8, 0.85, 0.9, or at least 0.95.

In certain embodiments, the signal peptide cleavage site is designed such that the predicted N-terminus is the N-terminus of the naturally existing protein. In other embodiments, the signal peptides and N terminus of the CRM197 are designed such that the N terminus is as close to the natively found N terminus as possible.

The natural N terminus after signal peptide cleavage of the CRM197 protein is GADDV...(Bell CE, Eisenberg D: Crystal structure of nucleotide-free diphtheria toxin. Biochemistry 1997, 36(3):481-488). In certain aspects of the disclosure, the N-terminus of CRM197 expressed in *E. coli* using the DsbA signal peptide can be:
MKKIWLALAGLVLAFS**A**S**A**-(cleavage)-ADDVVDSSK... and using the PelB signal peptide
MKKIWLALAGLVLAFS**AMA**-(cleavage)-GADDVVDSSKS....
Note the AXA motif at the cleavage site, where cleavage takes place after the second A.

Other cleavage sequences, and signal peptide cleavage site combinations are set forth in Table 2 below.

### 5.3 Expression Plasmids

A vast variety of expression vectors is known for recombinant expression in *E. coli* cells. In principle, any vector backbone can be used. Illustrative vectors are: pEC415 (Schulz H, Hennecke H, Thony-Meyer L: Prototype of a heme chaperone essential for cytochrome c maturation. Science 1998, 281(5380): 1197-1200), pBR322 (Bolivar F, Rodriguez RL, Greene PJ, Betlach MC, Heyneker HL, Boyer HW, Crosa JH, Falkow S: Construction and characterization of new cloning vehicles. II. A multipurpose cloning system. Gene 1977, 2(2):95-113), pBAD (Invitrogen corporation, Carlsbad, CA), pET series (Invitrogen), pUC series (Lin-Chao S, Chen WT, Wong TT: High copy number of the pUC plasmid results from a Rom/Rop-suppressible point mutation in RNA II. Mol Microbiol 1992, 6(22):3385-3393), pACT3, pEXT22, pEXT20 (Dykxhoorn DM, St Pierre R, Linn T: A set of compatible tac promoter expression vectors. Gene 1996, 177(1-2):133-136.), pBLUESCRIPT series (Stratagene, Agilent Technologies, Santa Clara, CA), pGEM series (Promega Corp., Madison, WI). All these vectors could be used for cloning the expression cassette of the preprotein under control of an inducible promoter.

Illustrative plasmids are provided as SEQ ID NOs: 1 and 2. The vector backbone is based on pBR322 containing a medium to high copy pMB1 origin of replication, an ampicillin resistance cassette which can be exchanged by a kanamycin cassette, the regulon of the araBAD operon encoding the AraC repressor and the araBAD promoter for high level protein expression induction.

In certain embodiments, CRM197 is expressed from chromosomally integrated constructs. This strategy requires additional technologies which are well known to those skilled in the art and would result in a genome-integrated expression construct consisting of the same elements as an expression plasmid but not requiring the selection cassette (only for selection upon genomic integration) and the origin of replication.

### 5.4 Promoters

Among well-known high expression inducible promoters, any can be used that is functional at the temperature for expression of the protein of interest. In certain embodiments, a promoter to be used with the methods provided herein is active below the temperature of 37°C, below 36°C, 35°C, 34°C, 33°C, 32°C, 31°C, or below 30°C. The following list contains illustrative bacterial expression promoters that can be used with the methods provided herein (Table 1):

**Table 1: Inducible promoters used in bacterial expression (Source: website of the: The Wolfson Centre for Applied Structural Biology of the Hebrew University of Jerusalem)**

| **Promoter** | **Source** | **Regulation** | **Induction** | **Level of Expression** | **Additional Information** |
|---|---|---|---|---|---|
| lac | *E. coli* | lacI, lacI^{q} * | IPTG | low | |
| lacUV5 | *E. coli* | lacI, lacI^{q} * | IPTG | low | Theoretically not subject to cAMP dependent regulation |
| tac (hybrid) | *E. coli* | lacI, lacI^{q} * | IPTG | Allows accumulation of protein to about 15-30% of total cell protein | Consists of the -35 region of the *trp* promoter and the -10 region of the *lac* promoter (differs from the *trc* promoter by 1 bp) |
| trc (hybrid) | *E. coli* | lacI, lacI^{q} * | IPTG | Allows accumulation of protein to about 15-30% of total cell protein | Consists of the -35 region of the *trp* promoter and the -10 region of the *lac* promoter (differs from the *tac* promoter by 1 bp) |
| trp | *E. coli* | Addition of fructose to the growth medium increases down regulation under non-induced conditions. | Tryptophan starvation or addition of B-indoleacrylic acid | | |
| *araBAD* | *E. coli* | araC | 1-arabinose | Weaker than the *tac* promoter | There is extensive heterogeneity in cell populations treated with subsaturating concentrations of 1-arabinose (some bacteria are fully induced and others not at all). |
| phoA | *E. coli* | phoB (positive) phoR (negative) | phosphate starvation | | Tightly controlled. Induction requires phosphate starvation, and so can limit the duration of protein synthesis. |
| recA | *E. coli* | lexA | nalidixic acid | | |
| proU | *E. coli* | | osmolarity | | |
| cst-1 | *E. coli* | | glucose starvation | | |
| tetA | *E. coli* | | tetracyclin | | |
| cadA | *E. coli* | cadR | pH | | |
| nar | *E. coli* | fhr | anearobic conditions | | |
| cspA | *E. coli* | | Thermal cold shock (shift to below 20°C) | | The cspA core promoter is only weakly induced by temperature downshift. A 159 nucleotide long untranslated region at the 5' end of cspA driven transcripts makes them highly unstable at 37°C and significantly increases their stability at low temps. This region also favors their engagement by cold modified translational machinery. The cspA system becomes repressed 1-2 hours after temperature downshift. |
| SP6 | Salmonella phage | | | | |
| T7 | T7 phage | cIts857 | thermal | | |
| T7-lac operator | T7 phage | lacI^{q} * | IPTG | Allows accumulation of protein to about 40-50% of total cell protein | |
| T3-lac operator | T3 phage | lacI^{q} * | IPTG | | |
| T5-lac operator | T5 phage | lacI, lacI^{q} * | IPTG | | This promoter is recognized by the *E*. *coli* RNA polymerase |
| T4 gene 32 | T4 phage | | T4 infection | | |
| nprM-lac operator | *Bacillus* | lacI^{q} * | IPTG | | |
| VHb | *Vitreoscilla* | | oxygen | | |

### 5.5 Culture Medium

Culture medium for protein production can be any defined, semi-defined or complex medium suitable for over-expression of recombinant proteins in *E. coli.* A rich complex medium like terrific broth (TB) is preferred, but defined mineral salts media may also be used. Terrific broth is composed of 24 g/l yeast extract, 12 g/l tryptone or peptone (i.e. proteolytically digested casein, soy protein or other protein), and 4% (v/v) glycerol. In addition, the medium is buffered.

In certain specific embodiments, the concentration of Magnesium ions is at most 10nM, 50nM, 100nM, 250nM, 500nM, 750nM, or at most 1mM. In certain specific embodiments, no Magnesium is added. In certain specific embodiments, no MgCl₂ is added to the culture medium.

In certain specific embodiments, the pH of the culture medium is between 6 and 9. In certain specific conditions, yeast extract can be present in the culture medium at a concentration of between 10-30 g/l. In certain specific embodiments, the culture medium comprises glycerol from 2.5% to 10%. In certain other embodiments, the culture medium comprises glycerol at least 5%, 10%, 15%, or at least 20%.

### 5.6 Induction and Expression

Expression cultures before induction can be grown at different temperatures, for example, temperatures ranging from 4-35°C or 18-37°C. In certain embodiments, expression cultures before induction are grown at a temperature within the range of 18-20°C, 20-22°C, 22-24°C, 24-26°C, 26-28°C, 28-30°C, 30-32°C, 32-34°C, or 34-36°C. In certain embodiments, expression cultures before induction are grown at a temperature of about 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, or 37°C.

Cultivation temperatures after induction can fall into certain ranges, for example, temperatures ranging from 4-35°C or 18-37°C, and can be different from the before induction conditions. For example, a pre-induction culture can be grown at higher temperatures, e.g., a temperature described above, and then shifted to a lower temperature, e.g., a temperature in the range of 15-30°C, for production. In certain embodiments, cultures after induction are grown at a temperature within the range of 18-20°C, 20-22°C, 22-24°C, 24-26°C, 26-28°C, 28-30°C, 30-32°C, 32-34°C, or 34-36°C. In a specific embodiment, said temperature falls within a range that is lower than the range at which the pre-induction culture is grown. In certain embodiments, cultures after induction are grown at a temperature of about 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, or 37°C. In a specific embodiment, said temperature falls within a range that is lower than the range at which the pre-induction culture is grown.

Depending on the construct, expression time can be from 2-20 hrs. Inducer concentrations are, dependent on the promoter, from 0.01 to 1 % (w/v) arabinose (ParaBAD), or from 10 to 1000µM IPTG. Induction can be done at OD600 values obtained during fermentation between 0.3 to 1.5 in shake flask cultures, and at OD600 between 5 to 200 in bioreactor fermentations. In certain specific embodiments, induction is done at an OD600 of between 5 and 50, 25 and 75, 50 and 100, 75 and 125, 100 and 150, 125 and 175, 150 and 200, or 175 and 200. In certain embodiments, induction is done at the beginning of the log phase in shake flask. Bioreactor fermentations may be done at constant pO2 values ranging from 0 % to 40%. pO2 regulation may be done by regulating stirrer speed or aeration rate.

In certain embodiments, the promoter is inducible with arabinose; arabinose concentrations can be at least 0.01, 0.05, 0.1, 0.2, 0.3, 0.4, 0.5, 0.6, 0.7, 0.8, 0.9, or at least 1 % (w/v) arabinose. In certain embodiments, concentration of the inducer arabinose is at most 0.01, 0.05, 0.1, 0.2, 0.3, 0.4, 0.5, 0.6, 0.7, 0.8, 0.9, or at most 1 % (w/v) arabinose

In certain embodiments, the promoter is inducible with IPTG; IPTG concentrations can be at least 10, 25, 50, 75, 100, 200, 250, 300, 400, 500, 600, 700, 750, 800, 900 or at least 1000 µM IPTG. In certain embodiments, concentration of the inducer IPTG is at most 10, 25, 50, 75, 100, 200, 250, 300, 400, 500, 600, 700, 750, 800, 900 or at most 1000 µM IPTG.

In certain embodiments, expression is performed in shake flask cultures. OD₆₀₀ values at the time of induction are at least 0.3, 0.4, 0.5, 0.6, 0.7, 0.8, 0.9, 1, 1.1, 1.2, 1.3, 1.4, or at least 1.5 in shake flask cultures. In certain embodiments, OD₆₀₀ values at the time of induction are at most 0.3, 0.4, 0.5, 0.6, 0.7, 0.8, 0.9, 1, 1.1, 1.2, 1.3, 1.4, or at most 1.5 in shake flask cultures.

In certain embodiments, expression is performed in bioreactor fermentations. OD600 values at the time of induction are at least 5, 10, 15, 20, 25, 50, 75, or at least 100 in bioreactor fermentations. In certain embodiments, OD₆₀₀ values at the time of induction are at most 5, 10, 15, 20, 25, 50, 75, or at most 100 in bioreactor fermentations.

Bioreactor fermentations can be performed at constant pO2 values of at least 1%, 2%, 3%, 4%, 5%, 6%, 7%, 8%, 9%, 10%, 15%, 20%, 25%, 30%, 35%, or at least 40%. In certain embodiments, bioreactor fermentations can be performed at constant pO2 values of at most 1%, 2%, 3%, 4%, 5%, 6%, 7%, 8%, 9%, 10%, 15%, 20%, 25%, 30%, 35%, or at most 40%.

### 5.7 Host Cells

Expression strains for recombinant production of the target protein can be but are not limited to *E. coli* K12 and B strains, like W3110, DB1, DH5a, BL21, BL21(DE3), C43, JM109, JM101, JM110, and derivatives thereof (Huang CJ, Lin H, Yang X: Industrial production of recombinant therapeutics in E. coli and its recent advancements. Journal of Industrial Microbiology & Biotechnology 2012, 39(3):383-399). Host cells may be chromosomally modified to accommodate optimal expression of the CRM197 protein. For example, periplasmic proteases like DepP, Prc, Spr, and/or protease III may be deleted in production strains. Deletions may be useful alone or in combinations with other proteases. In addition, suppressor mutations like for example sprW148R (Chen C, Snedecor B, Nishihara JC, Joly JC, McFarland N, Andersen DC, Battersby JE, Champion KM: High-level accumulation of a recombinant antibody fragment in the periplasm of E. coli requires a triple-mutant (degP prc spr) host strain. Biotechnology and bioengineering 2004, 85(5):463-474.) may increase CRM197 protein yield.

### 5.8 Assays

Methods to characterize yield, purity, stability, nicking degree, toxicity, endotoxin content are well established and define the quality for use of CRM197 in a vaccine. Analysis of CRM197 is done by, e.g., high performance size exclusion chromatography, isoelectric focusing, SDS-PAGE and Western Blot, molecular weight determination by MS, N terminal sequencing, amino acid analysis, reverse phase liquid chromatography, electrospray mass spectroscopy, and peptide mapping by mass spectroscopy after tryptic digestion.

Analytical methods are described and parameters that define acceptable quality are well established for use in medicinal products. Detailed information and framework parameters are given e.g., in the guidelines released by the European medicinal agency, EMEA and can be found at EMEA's website, e.g., for the CRM197-containing vaccine Prevenar.

### 5.8.1 Concentration of Expression Product

Standard protein concentration technologies like the Lowry assay, BCA assay, and Bradford assays could be used, as well as determination of the UV absorption at 280 nm and quantification from Coomassie stained SDS-PAGE gels by densitometry or capillary gel electrophoresis by fluorescent dye intensity measurements.

### 5.8.2 Folding of Expression Product

Folding of the product can be analyzed directly by circular dichroism spectroscopy, protein NMR spectroscopy, and HPSEC. Indirect methods include solubility measurement, protease resistance, and activity assays for toxicity in the case of the DT A fragment, and binding assays for the CRM197 and DT B fragments.

### 5.8.3 Inclusion Bodies of Expression Product

Inclusion body formation is easily quantified by first homogenization of the harvested cells after fermentation, low spin centrifugation for sedimentation of the insoluble matter, and comparing pellet and supernatant side by side in an equivalent optical density manner. Intensity of the protein band allows estimation of the proportion in supernatant (soluble protein) and pellet (insoluble aggregates and inclusion bodies).

### 5.8.4 Solubility of Expression Product

Supernatant solution containing the protein can be centrifuged and sterile filtered. If the protein remains in solution and is not depleted from the filtrate and supernatants, the protein is soluble. A more sophisticated method is dynamic light scattering. It allows the determination of particle size, which is indicative of the oligomeric or micro aggregated state of the purified protein.

Solubility is inversely proportional to aggregate formation such that a finding of high solubility demonstrates no or low level of aggregate formation.

### 5.8.5 Periplasmic Localization of Expression Product

Periplasmic localization is measured by fractionation of cells and comparing specific protein yields observed in periplasm and spheroplast fractions. Fractionation is done by using the sucrose-lysozyme method (Kowarik M, Young NM, Numao S, Schulz BL, Hug I, Callewaert N, Mills DC, Watson DC, Hernandez M, Kelly JF et al: Definition of the bacterial N-glycosylation site consensus sequence. The EMBO journal 2006, 25(9):1957-1966), osmotic shock (Johansson HJ, Jagersten C, Shiloach J: Large scale recovery and purification of periplasmic recombinant protein from E. coli using expanded bed adsorption chromatography followed by new ion exchange media. J Biotechnol 1996, 48(1-2):9-14.), or polymyxin (Schulz H, Hennecke H, Thony-Meyer L: Prototype of a heme chaperone essential for cytochrome c maturation. Science 1998, 281(5380): 1197-1200). Fraction aliquots are normalized based on sample volumes and culture OD₆₀₀ and analyzed by SDS PAGE and Western blotting.

### 5.8.6 Cleavage of Signal Sequence

Cleavage of signal peptides is assayed by i) gel shift analysis of fractionated cells as described in 5.8.5. In this analysis, unprocessed pre-protein may be accumulating in cytoplasmic aggregates or membranes, and processed protein will be present in soluble, periplasmic fractions. Different electrophoretic mobility will constitute a shift between processed and unprocessed pre-proteins by SDS-PAGE (and Western blot if necessary). Eventually, N-terminal amino acid sequencing can be used to determine the processed N-terminus and thereby define the cleavage site experimentally.

### 5.8.7 Potential toxicity of CRM197

CRM197 can be tested for the presence of active toxin by measuring the ADP-ribosyl transferase activity. Additionally, other tests (cytotoxicity in HeLa cells or Vero cells in vitro, lethality in guinea pigs in vivo, abnormal toxicity test) can be used to demonstrate the nontoxicity of CRM197

### 5.9 Compositions

CRM197, produced in accordance with the methods provided herein can be further processed to immunogenic compositions or vaccines. For example, the protein can be conjugated to an oligosaccharide or a polysaccharide to yield an immunogenic composition or vaccine. In certain, specific embodiments, such an immunogenic composition or vaccine has improved immunogenic properties over prior art compositions. Without being bound by theory, the methods provided herein provide a more homogenous population of soluble CRM197 protein. As such any immunogenic composition or vaccine is more effective than prior art compositions.

In certain embodiments, provided herein is a composition comprising CRM197, that has been produced in accordance with the methods provided herein. In certain more specific embodiments, such a composition is a pharmaceutical composition. Even more specifically, such a pharmaceutical composition further comprises and pharmaceutically acceptable carrier.

### 6 EXAMPLES

### 6.1 Example 1

Different experimental setups were tested and the CRM197 yield was determined by Western blotting using anti diphtheria toxin antiserum for detection of CRM197.

A DNA open reading frame for CRM197 expression was synthesized by a commercial provider (Genescript, Piscataway, NJ) in a codon optimized fashion containing the N-terminal signal peptide of the DsbA protein of *E. coli* instead of the natural signal peptide, and a C terminal hexa-histidine tag. The resulting protein sequence is SEQ ID 5. The open reading frame for ssDsbA-CRM197-his6 was inserted into the NdeI and XbaI sites of pEC415 (Schulz H, Hennecke H, Thony-Meyer L: Prototype of a heme chaperone essential for cytochrome c maturation. Science 1998, 281(5380):1197-1200).

From this plasmid, various mutants were made to analyze the differences of the CRM197 yields. Mutations were introduced at the expected signal peptide cleavage site by quick change mutagenesis as described by the manufacturer (Stratagene, Agilent Technologies, Santa Clara, CA). The resulting constructs are summarized in Table 1.

The mentioned plasmids were transformed into BL21 and W3110 cells to perform protein expression experiments. Transformed colonies were picked from an LB plate and used to inoculate LB medium liquid culture, which were grown over night at 37°C. The high density cultures were diluted to an OD₆₀₀ of 0.05 into fresh LB medium and grown further until the OD reached a value of OD₆₀₀=0.5. Then arabinose was added for induction of recombinant protein expression. Initial experiments using some of the mentioned constructs were performed under various conditions.

However, no CRM197 protein was detected in cellular extracts when compared to control cells expressing no protein or expressing EPA (Ihssen J, Kowarik M, Dilettoso S, Tanner C, Wacker M, Thony-Meyer L: Production of glycoprotein vaccines in E. coli. Microbial cell factories 2010, 9:61). Neither at 30 nor 37°C, using overnight induction times and LB medium supplemented with ampicillin for plasmid maintenance.

Subsequently, expression was conducted as follows. For the expression, high density cultures from overnight incubations were diluted into terrific broth for better cell viability. Cultures were grown until exponential phase and induced for 2 hours and overnight, and then cells were harvested and cellular extracts prepared by dissolving OD equivalent amounts of biomass in Lämmli sample buffer. The extracts were separated by SDS PAGE and electrotransferred to nitrocellulose membranes for subsequent immunodetection using anti DT and anti his tag antisera. Surprisingly, a protein signal at the expected electrophoretic mobility of CRM197 at about 60 kDa was detected after 2 hours of induction. Expression constructs p932, p934, and p722 led to detectable signals in anti DT and anti his tag antiserum immunoblots. p932 appeared to produce most, p934 less, and p722 even lesser CRM197 signals. A control extract from cells containing an expression plasmid lacking a signal peptide sequence showed CRM197 at the correct molecular weight range and confirmed the identity of the material in the other lanes.

These experiments showed that CRM197 could be expressed, but not whether it was soluble or folded. As indicated in Fig. 1, CRM197 without a signal peptide was detected and expected in cytoplasmic inclusion bodies. The expected yields are unknown and can only be estimated by comparison to the expression of EPA. In this comparison, CRM197 reaches yields similar to EPA based on the signal intensities observed using anti his tag antiserum Western blotting as illustrated in Fig. 1 (compare lanes 4A and 4B to 5A and 5B). EPA in controlled bioreactor fermentations leads to up to 0.5 g/l protein.

The order of efficiency for CRM197 production was p932>p934>p722. The methionine residue encoded in the cleavage site from the CRM197 expressed from p722 may interfere with productivity, and also the glycine residue has some influence. It appears, however, that formation of an N terminus of CRM197 with one amino acid less (ADDV...; p932) than the natural N terminus in combination with the serine residue at the -2 position relative to the cleavage site leads to the optimal expression context when the DsbA signal peptide is used.

However, it was possible to detect CRM197 signals in expression experiments using different expression constructs and TB medium.

### 6.2 Example 2

To analyze solubility and overall yield in shake flasks, CRM197 was purified from cell cultures using two different expression constructs. Expression strain was BL21, the expression plasmids p932 or p933. 5 liter shake flasks containing 1 1 TB supplemented with ampicillin were inoculated with a pre-culture grown in LB supplemented with ampicillin and cultured at 30°C. At OD₆₀₀ of 0.5, arabinose was added to 0.2 % (w/v) and expression allowed for 2 (p932) or 4 hours (p933). Cells were then harvested by centrifugation, resuspended in buffer for periplasma extraction (20% w/v sucrose, 30 mM Tris·HCl pH 8.0, 1 mM EDTA, 1 mg/ml lysozyme, 1 tablet / 80 mL Complete protease inhibitor mix (Roche, Basel, Switzerland)) at a ratio of 20 OD per ml, incubated on ice for 30 min, and centrifuged for 15 min at 8000 rpm and 4°C. The supernatant was further treated with DNase (Fluka, Balgach, Switzerland), centrifuged at 4°C, and the supernatant sterile filtered. The filtrate was prepared for purification using Ni2+ affinity chromatography. Load, wash and elution were performed at specific imidazole concentrations (10, 20, 500 mM). Elution fractions were analyzed by SDS PAGE and Coomassie brilliant blue staining (Fig. 2).

A major band corresponding to CRM197 was detected in elution fractions from the purification. Protein determination resulted in values of about 2 mg protein from construct p932, and about 4 mg from construct p933 per liter fermentation broth. N terminal sequencing and MALDI MSMS of excised protein bands from this SDS PAGE gel confirmed the N terminus of CRM197 in both cases (see Table 1) and that the protein is indeed CRM197.

The difference between protein expressed from p932 and p933 is the signal peptide sequence and the resulting mature N-terminus of CRM197. p933 produced the correct wild type N terminus; although the Y score for cleavage efficiency is lower than for p932. In figure 1, p934 borne expression appears to be even less efficient, and accordingly, the Y score is less. Thus a combination of a high Y score value and a signal peptide cleavage position resulting in the native N-terminus GADDV seems to be the optimal configuration for high yield CRM197 expression in *E. coli.* Expression time, temperature, medium and inducer concentration may influence signal peptide cleavage yield, speed, and efficiency and accordingly CRM197 yields.

### 6.3 Example 3

To analyze the productivity of different constructs in parallel, small scale shake flask expression experiments were performed, the periplasmic extracts were prepared and analyzed by SDS PAGE for the CRM197 band intensity by Coomassie staining (Fig. 3) and quantified (Table 2). The detailed expression conditions are given in the legend of Fig. 3 and in Table 2.

DsbA, MalE, and PelB signal peptides resulted in the best yields in combination with optimized expression conditions. The expression conditions had a stronger influence on yields than the signal peptide cleavage site configurations. However, the importance of signal peptide cleavage site sequence is shown e.g., by the low yields obtained with the p722 expression plasmid (at 25°C). Although p722 encodes the DsbA signal, the yield is low compared to other sequences (encoded in e.g., p932, p933, p934, or p936). Signal peptide cleavage site configurations can be classified according to their yield efficiencies: ASA-ADD and AMA-GADD appear better than ASA-GADD, and AMG-ADD being the least efficient site. Y scores do not correlate with expression levels.

All tested constructs containing the PelB signal resulted in high yields at 30°C expression temperature. Differences in the signal peptide cleavage site sequence did not drastically influence yields. However, the differences in signal peptide cleavage site sequence were small in this set of constructs.

**Table 2 Plasmids and signal peptides used for periplasmic expression of CRM197 and a well-secreted reference protein (EPA-6H). Plasmids were transformed in E. coli (W3110 derived strain) and cultivated in TB medium at temperatures yielding the highest levels of recombinant proteins. Samples for preparation of periplasmic extracts were taken 4 h after induction with 4 g L⁻¹ L-arabinose, concentration of resuspended cells in sucrose-lysozyme extraction buffer was normalized to OD₆₀₀ = 20. Concentrations of overexpressed recombinant proteins in periplasmic extracts were estimated by image analysis of a Coomassie-stained SDS-PAGE gel using marker bands at 55 kDa as reference. Protein yields in shake flasks were back calculated via OD₆₀₀ at the time of sampling. n.a.: not analyzed.**

| **Plasmid No.** | **Name GVXN** | **Protein SEQ ID** | **Signal peptide** | **Predicted cleavage site** | **Cleavage probability** (Y score Signal P 4.0) | **Determined N-terminus after export** | **C-terminal 6xHis tag** | **Optimal expression temperature** | **Protein conc. (CRM197/EPA) in periplasmic extracts** (µg mL⁻¹) | **OD₆₀₀ at sampling** | **Protein yield in shake flask** (mg L⁻¹) |
|---|---|---|---|---|---|---|---|---|---|---|---|
| Plasmids for periplasmic expression of CRM197, backbone pEC415 | | | | | | | | | | | |
| 1 | p722 | 5 | DsbA | AFSAMG-ADDV | 0.763 | n.a. | yes | 25°C | 24 | 1.28 | 1.6 |
| 2 | p932 | 2 | DsbA | AFSASA-ADDV | 0.878 | ADDV... | yes | 25°C | 66 | 2.66 | 8.8 |
| 3 | p933 | 4 | DsbA | AFSAMA-GADDV | 0.783 | GADDV... | yes | 25°C | 51 | 1.30 | 3.3 |
| 4 | p936 | x | DsbA | AFSAMA-GADDV | 0.783 | n.a. | no | 20-25°C | 57 | 1.62 | 4.6 |
| 5 | p934 | x | DsbA | AFSASA-GADDV | 0.681 | n.a. | yes | 25°C | 64 | 1.38 | 4.4 |
| 6 | p1027 | x | MalE | SASALA-MGADDV | 0.722 | n.a. | yes | 25°C | 97 | 1.43 | 6.9 |
| 7 | p1029 | x | MalE | SASALA-ADDV | 0.894 | n.a. | yes | 25°C | 93 | 1.54 | 7.1 |
| 8 | pl030 | x | OmpA | ATVAQA-M GADDV | 0.790 | n.a. | yes | 25°C | 9 | 1.53 | 0.7 |
| 9 | pl032 | x | OmpA | ATVAQA-ADDV | 0.898 | n.a. | yes | 25°C | 11 | 1.54 | 0.9 |
| 10 | p1033 | x | PelB | AQPAMA-MGADDV | 0.878 | n.a. | yes | 30°C | 36 | 3.29 | 5.9 |
| 11 | p1018 | x | PelB | AQPAMA-GADDV | 0.874 | n.a. | yes | 30°C | 27 | 3.72 | 5.0 |
| 12 | p1035 | x | PelB | AQPAMA-ADDV | 0.874 | n.a. | no | 30°C | 32 | 3.96 | 6.4 |
| 13 | p1036 | x | PelB | AQPAMA-AGADDV | 0.918 | n.a. | yes | 30°C | 44 | 3.27 | 7.2 |
| 14 | p938 | x | LT-IIb* | SVQAHA-GADDV | 0.885 | n.a. | yes | 30°C | 13 | 1.11 | 0.7 |
| 15 | p1039 | x | XynA | SATASA-MGADDV | 0.464 | n.a. | yes | 25°C | 13 | 0.94 | 0.6 |

| Reference plasmid for periplasmic expression of EPA, backbone pEC415 | | | | | | | | | | | |
|---|---|---|---|---|---|---|---|---|---|---|---|
| 16 | p150 | - | DsbA | AFSASA-AEEA | 0.873 | AEEA... | yes | 30°C | 73 | 2.58 | 9.4 |

| | | | | | | | | | | | |
|---|---|---|---|---|---|---|---|---|---|---|---|
| * *E. coli* heat-labile enterotoxin type IIb, chain B | | | | | | | | | | | |

### 7 SEQUENCES

### SEQ ID 3: Signal peptide containing Crm197 amino acid sequence expressed from p932

### SEQ ID 4: Signal peptide containing Crm197 amino acid sequence expressed from p933

### SEQ ID 5: translated protein sequence of p722

### SEQ ID 6: mature, secreted CRM197

## Claims

1. A method of producing CRM197 wherein the method comprises culturing a host cell comprising a nucleic acid encoding CRM197 wherein CRM197 is fused to a heterologous signal peptide that targets CRM197 to the periplasm of the host cell, wherein the nucleic acid encodes a cleavage site between the signal peptide that targets CRM197 to the periplasm and the CRM197 protein wherein the cleavage site comprises the amino acid sequence aa1-aa2-aa3-(cleavage site)-aa4-aa5-aa6-aa7-aa8, wherein
aa1 is Ala, Ser, Gly, Cys, Thr or Gln;
aa2 is selected from any natural amino acid;
aa3 is selected from any natural amino acid except Phe, His, Tyr, Trp, Asp, Glu, Lys, Arg, Asn and Gln;
aa4 to 8 is selected from ala-asp-asp-val and met-gly-ala-asp;
or wherein the cleavage site comprises the amino acid sequence aa1-aa2-aa3-(cleavage site)-aa4-aa5-aa6-aa7-aa8, wherein
aa4 to 8 is selected from ala-asp-asp-val and met-gly-ala-asp; and wherein the first 70 aa of the first open reading frame results in a Y score when analyzed by SignalP 4.0 Server of more than 0.72.

2. The method of claim 1 wherein the wild type signal peptide of CRM197 has been deleted.

3. The method of claim 1 wherein the wild type signal peptide of CRM197 has been replaced by the heterologous signal peptide.

4. The method of claim 1 wherein the heterologous signal peptide is selected from the group consisting of the signal peptide from *E. coli* heat-labile enterotoxin, *E. coli* outer membrane porin (OmpA), *E. coli* maltose binding protein (MalE), *E. carotovorans* pectate lyase (PelB), and *Bacillus* sp. endoxylanase (XynA).

5. The method of claim 1 wherein CRM197 is produced at a concentration of at least 5, 10, 25, 50, 75, 100, 125, 125, 150, 175, 200, 225, 250, 300, 400, 500, 600, 700, 800, 900, or at least 1000 mg protein per liter culture medium.

6. The method of claim 1 or 5 wherein at least 50% of the produced protein is properly folded as determined by circular dichroism.

7. The method of claim 1 or 5 wherein at least 50% of the produced protein is not present in aggregates.

8. The method of claim 1 or 5 wherein at least 50% of the produced protein is soluble.

9. The method of claim 1 or 5 wherein the heterologous nucleotide sequence encodes a cleavage site between the signal peptide that targets CRM197 to the periplasm and the CRM197 protein wherein the cleavage site comprises the amino acid sequence aa1-aa2-aa3-(cleavage site)-aa4-aa5-aa6-aa7-aa8, wherein
aa1 is Ala;
aa2 is selected from any natural amino acid;
aa3 is Ala;
aa4 to 8 is selected from ala-asp-asp-val and met-gly-ala-asp.

10. The method of claim 1 or 5 wherein the heterologous nucleotide sequence encodes the protein of SEQ ID NO: 1 or 2.

11. The method of claim 1 or 5 wherein the heterologous nucleotide sequence is operatively linked to a promoter selected from the group consisting of the 1-arabinose inducible araBAD promoter (PBAD), the lac promoter, the 1-rhamnose inducible rhaP BAD promoter, the T7 RNA polymerase promoter, the trc and tac promoter, the lambda phage promoter p L , and the anhydrotetracycline-inducible tetA promoter/operator.

12. The method of claim 1 or 5 wherein the nucleic acid encoding CRM197 is inserted in a high copy expression plasmid, optionally the high copy expression plasmid is pEC415, pBR322, pBAD, pET series, pUC series, pACT3, pEXT22, pEXT20, pBLUESCRIPT series, pGEM series.

13. The method of claim 1 or 5 wherein the expression of CRM197 is induced at a culture density of OD600 >0.3.

14. The method of claim 1 or 5 wherein CRM197 is expressed at a temperature of 20, 25, 30, 32, 35°C or 37°C.

15. The method of claim 1 or 5 wherein at least 50% of the expressed CRM197 have an N-terminus of ADDV or MGADDV.

16. The method of claim 1 or 5 wherein at least 50% of the expressed CRM197 have a disulfide bond between Cys186 and Cys201.

## Patentansprüche

1. Verfahren zur Produktion von CRM197, wobei das Verfahren Kultivieren einer Wirtszelle, welche eine CRM197-codierende Nukleinsäure umfasst, umfasst, wobei CRM197 an ein heterologes Signalpeptid, welches CRM197 in das Periplasma der Wirtszelle zielt, fusioniert ist, wobei die Nukleinsäure eine Spalt-Stelle (cleavage site) zwischen dem Signalpeptid, welches CRM197 in das Periplasma zielt, und dem CRM197-Protein codiert, wobei die Spalt-Stelle die Aminosäuresequenz aa1-aa2-aa3-(Spalt-Stelle)-aa4-aa5-aa6-aa7-aa8 umfasst, wobei
aa1 Ala, Ser, Gly, Cys, Thr oder Gln ist;
aa2 ausgewählt ist aus irgendeiner natürlichen Aminosäure;
aa3 ausgewählt ist aus irgendeiner natürlichen Aminosäure außer Phe, His, Tyr, Trp, Asp, Glu, Lys, Arg, Asn und Gln;
aa4 bis 8 ausgewählt ist aus ala-asp-asp-val und met-gly-ala-asp;
oder wobei die Spalt-Stelle die Aminosäuresequenz aa1-aa2-aa3-(Spalt-Stelle)-aa4-aa5-aa6-aa7-aa8 umfasst,
wobei
aa4 bis 8 ausgewählt ist aus ala-asp-asp-val und met-gly-ala-asp; und wobei die ersten 70 Aminosäuren (aa) des ersten offenen Leserahmens in einem Y-Wert, wenn mittels SignalIP 4.0-Server gemessen, von mehr als 0,72 resultiert.

2. Verfahren gemäß Anspruch 1, wobei das Wildtyp-Signalpeptid von CRM197 deletiert wurde.

3. Verfahren gemäß Anspruch 1, wobei das Wildtyp-Signalpeptid von CRM197 durch das heterologe Signalpeptid ersetzt wurde.

4. Verfahren gemäß Anspruch 1, wobei das heterologe Signalpeptid ausgewählt wird aus der Gruppe bestehend aus dem Signalpeptid von hitzelabilem *E. coli-*Enterotoxin, *E. coli*-äußerem Membranporin (OmpA), *E*. *coli*-Maltose-bindendem Protein (MalE), *E. carotovorans* Pectat-Lyase (PelB) und *Bacillus sp*.-Endoxylanase (XynA).

5. Verfahren gemäß Anspruch 1, wobei CRM197 mit einer Konzentration von mindestens 5, 10, 25, 50, 75, 100, 125, 125, 150, 175, 200, 225, 250, 300, 400, 500, 600, 700, 800, 900 oder mindestens 1000 mg Protein pro Liter Kulturmedium produziert wird.

6. Verfahren gemäß Anspruch 1 oder 5, wobei mindestens 50% des produzierten Proteins richtig gefaltet ist, wie durch circulären Dichroismus festgestellt.

7. Verfahren gemäß Anspruch 1 oder 5, wobei mindestens 50% des produzierten Proteins nicht in Aggregaten vorliegt.

8. Verfahren gemäß Anspruch 1 oder 5, wobei mindestens 50% des produzierten Proteins löslich ist.

9. Verfahren gemäß Anspruch 1 oder 5, wobei die heterologe Nukleinsäuresequenz eine Spalt-Stelle zwischen dem Signalpeptid, welches CRM197 in das Periplasma zielt, und dem CRM197-Protein codiert, wobei die Spalt-Stelle die Aminosäuresequenz aa1-aa2-aa3-(Spalt-Stelle)-aa4-aa5-aa6-aa7-aa8 umfasst, wobei
aa1 Ala ist;
aa2 ausgewählt ist aus irgendeiner natürlichen Aminosäure;
aa3 Ala ist;
aa4 bis 8 ausgewählt ist aus ala-asp-asp-val und met-gly-ala-asp;

10. Verfahren gemäß Anspruch 1 oder 5, wobei die heterologe Nukleinsäuresequenz das Protein von SEQ ID NO: 1 oder 2 codiert.

11. Verfahren gemäß Anspruch 1 oder 5, wobei die heterologe Nukleinsäuresequenz operabel mit einem Promotor ausgewählt aus der Gruppe bestehend aus dem L-Arabinoseinduzierbarem araBAD Promotor (PBAD), dem Lac-Promotor, dem L-Rhamnose-induzierbaren rhaP BAD Promotor, dem T7-RNA-Polymerase-Promotor, dem Trc und Tac Promotor, dem Lambda-Phagen-Promotor p L, und dem Anhydrotetracyclininduzierbaren tetA Promotor/Operator, verbunden ist.

12. Verfahren gemäß Anspruch 1 oder 5, wobei die CRM197-codierende Nukleinsäure in ein Hochkopienzahl-Plasmid eingefügt ist, optional ist das Hochkopienzahl-Plasmid pEC415, pBR322, pBAD, pET-Serie, pUC-Serie, pACT3, pEXT22, pEXT20, pBLUESCRIPT-Serie, pGEM-Serie.

13. Verfahren gemäß Anspruch 1 oder 5, wobei die Expression von CRM197 bei einer Kulturdichte von OD600 > 0,3 induziert wird.

14. Verfahren gemäß Anspruch 1 oder 5, wobei CRM197 bei einer Temperatur von 20, 25, 30, 32, 35 °C oder 37 °C exprimiert wird.

15. Verfahren gemäß Anspruch 1 oder 5, wobei mindestens 50% des exprimierten CRM197 einen N-Terminus von ADDV oder MGADDV hat.

16. Verfahren gemäß Anspruch 1 oder 5, wobei mindestens 50% des exprimierten CRM197 eine Disulfid-Brücke zwischen Cys186 und Cys201 hat.

## Revendications

1. Procédé de production de CRM197 dans lequel le procédé comprend la culture d'une cellule hôte comprenant un acide nucléique codant pour CRM197, dans lequel CRM197 est fusionnée à un peptide signal hétérologue qui cible CRM197 sur le périplasme de la cellule hôte, dans lequel l'acide nucléique code pour un site de clivage entre le peptide signal qui cible CRM197 sur le périplasme et la protéine CRM197, dans lequel le site de clivage comprend la séquence d'acides aminés aa1-aa2-aa3-(site de clivage)-aa4-aa5-aa6-aa7-aa8, où
aa1 est Ala, Ser, Gly, Cys, Thr ou Gln ;
aa2 est choisi parmi tout acide aminé naturel ;
aa3 est choisi parmi tout acide aminé naturel à l'exception de Phe, His, Tyr, Trp, Asp, Glu, Lys, Arg, Asn et Gln ;
aa4 à 8 est choisi parmi ala-asp-asp-val et met-gly-ala-asp ;
ou dans lequel le site de clivage comprend la séquence d'acides aminés aa1-aa2-aa3-(site de clivage)-aa4-aa5-aa6-aa7-aa8, où
aa4 à 8 est choisi parmi ala-asp-asp-val et met-gly-ala-asp ; et dans lequel les 70 premiers aa du premier cadre de lecture ouvert ont pour résultat un score Y, lorsqu'ils sont analysés par SignalP 4.0 Server, de plus de 0,72.

2. Procédé selon la revendication 1, dans lequel le peptide signal de type sauvage de CRM197 a été supprimé.

3. Procédé selon la revendication 1, dans lequel le peptide signal de type sauvage de CRM197 a été remplacé par le peptide signal hétérologue.

4. Procédé selon la revendication 1, dans lequel le peptide signal hétérologue est choisi parmi le groupe constitué du peptide signal de l'entérotoxine thermolabile de *E. coli,* de la porine de membrane externe de *E. coli* (OmpA), de la protéine de liaison au maltose de *E. coli* (MalE), de la pectate lyase de *E carotovorans* (PelB), et de l'endoxylanase de *Bacillus* sp. (XynA).

5. Procédé selon la revendication 1, dans lequel CRM197 est produite à une concentration d'au moins 5, 10, 25, 50, 75, 100, 125, 125, 150, 175, 200, 225, 250, 300, 400, 500, 600, 700, 800, 900, ou d'au moins 1 000 mg de protéine par litre de milieu de culture.

6. Procédé selon la revendication 1 ou 5, dans lequel au moins 50 % de la protéine produite est correctement repliée, comme déterminé par dichroïsme circulaire.

7. Procédé selon la revendication 1 ou 5, dans lequel au moins 50 % de la protéine produite n'est pas présente dans des agrégats.

8. Procédé selon la revendication 1 ou 5, dans lequel au moins 50 % de la protéine produite est soluble.

9. Procédé selon la revendication 1 ou 5, dans lequel la séquence nucléotidique hétérologue code pour un site de clivage entre le peptide signal qui cible CRM197 sur le périplasme et la protéine CRM197, dans lequel le site de clivage comprend la séquence d'acides aminés aa1-aa2-aa3-(site de clivage)-aa4-aa5-aa6-aa7-aa8, où
aa1 est Ala ;
aa2 est choisi parmi tout acide aminé naturel ;
aa3 est Ala ;
aa4 à 8 est choisi parmi ala-asp-asp-val et met-gly-ala-asp.

10. Procédé selon la revendication 1 ou 5, dans lequel la séquence nucléotidique hétérologue code pour la protéine ayant la SEQ ID NO : 1 ou 2.

11. Procédé selon la revendication 1 ou 5, dans lequel la séquence nucléotidique hétérologue est liée de manière opérationnelle à un promoteur choisi parmi le groupe constitué du promoteur araBAD inductible par 1-arabinose (PBAD), du promoteur lac, du promoteur rhaP BAD inductible par 1-rhamnose, du promoteur T7 d'ARN polymérase, du promoteur trc et tac, du promoteur p L du phage lambda, et du promoteur/opérateur tetA inductible par anhydrotétracycline.

12. Procédé selon la revendication 1 ou 5, dans lequel l'acide nucléique codant pour CRM197 est inséré dans un plasmide à grand nombre de copies, le plasmide à grand nombre de copies étant facultativement pEC415, pBR322, pBAD, série de pET, série de pUC, pACT3, pEXT22, pEXT20, série de pBLUESCRIPT, série de pGEM.

13. Procédé selon la revendication 1 ou 5, dans lequel l'expression de CRM197 est induite à une densité de culture de OD600 > 0,3.

14. Procédé selon la revendication 1 ou 5, dans lequel CRM197 est exprimée à une température de 20, 25, 30, 32, 35 °C ou 37 °C.

15. Procédé selon la revendication 1 ou 5, dans lequel au moins 50 % de la CRM197 exprimée ont une terminaison-N de ADDV ou MGADDV.

16. Procédé selon la revendication 1 ou 5, dans lequel au moins 50 % de la CRM197 exprimée ont une liaison disulfure entre Cys186 et Cys201.
